(19) Europäisches Patentamt
European Patent Office
Office européen des brevets

(11) **EP 2 861 980 B1**

(12) **FASCICULE DE BREVET EUROPEEN**

(45) Date de publication et mention de la délivrance du brevet:
**23.10.2019 Bulletin 2019/43**

(51) Int Cl.:
***G01N 33/00*** *(2006.01)* ***G06Q 30/02*** *(2012.01)*

(21) Numéro de dépôt: **13729939.2**

(22) Date de dépôt: **14.06.2013**

(86) Numéro de dépôt international:
**PCT/EP2013/062388**

(87) Numéro de publication internationale:
**WO 2013/189852 (27.12.2013 Gazette 2013/52)**

(54) **PROCEDE DE TRANSCRIPTION D'UNE ODEUR OU D'UN AROME EN UNE INFORMATION COLORÉE ET D'UNE INFORMATION COLORÉE EN UNE LISTE DE MOLECULES**

VERFAHREN ZUM ÜBERTRAGEN EINES GERUCHES ODER AROMAS IN FARBINFORMATIONEN UND VON FARBINFORMATIONEN IN EINE MOLEKÜLELISTE

METHOD FOR TRANSCRIBING AN ODOR OR AROMA INTO A COLOR INFORMATION AND A COLOR INFORMATION INTO A LIST OF MOLECULES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.06.2012 FR 1255688**

(43) Date de publication de la demande:
**22.04.2015 Bulletin 2015/17**

(73) Titulaire: **Université de Lorraine
54052 Nancy Cedex (FR)**

(72) Inventeurs:
• **JACQUOT, Muriel**
**F-54110 Rosières aux Salines (FR)**
• **MARIC, Yelena**
**F-54000 Nancy (FR)**
• **LAMBERT, Thierry**
**F-54600 Villers-les-Nancy (FR)**

(74) Mandataire: **Derval, Estelle et al
Marks & Clerk France
Conseils en Propriete Industrielle
Immeuble Visium
22, avenue Aristide Briand
94117 Arcueil Cedex (FR)**

(56) Documents cités:
**US-A1- 2008 010 146    US-A1- 2009 271 003**

• **HARUKA MATSUKURA ET AL: "Smelling screen: Technique to present a virtual odor source at an arbitrary position on a screen", PROCEEDINGS OF THE 2012 IEEE VIRTUAL REALITY WORKSHOP, 8 mars 2012 (2012-03-08), pages 127-128, XP032163093, DOI: 10.1109/VR.2012.6180915 ISBN: 978-1-4673-1247-9**
• **PENZA M ET AL: "Application of principal component analysis and artificial neural networks to recognize the individual VOCs of methanol/2-propanol in a binary mixture by SAW multi-sensor array", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER S.A, SWITZERLAND, vol. 89, no. 3, 1 avril 2003 (2003-04-01), pages 269-284, XP004414880, ISSN: 0925-4005, DOI: 10.1016/S0925-4005(03)00002-9**
• **YU-JIN KIM: "Can eyes smell? cross-modal correspondences between color hue-tone and fragrance family", COLOR RESEARCH & APPLICATION, vol. 38, no. 2, 31 octobre 2011 (2011-10-31), pages 139-156, XP055055930, ISSN: 0361-2317, DOI: 10.1002/col.20717**
• **YELENA MARIC ET AL: "Contribution to understanding odour-colour associations", FOOD QUALITY AND PREFERENCE, vol. 27, no. 2, 11 mai 2012 (2012-05-11), pages 191-195, XP055055931, ISSN: 0950-3293, DOI: 10.1016/j.foodqual.2012.05.001**

• FU ET AL: "A pattern recognition method for electronic noses based on an olfactory neural network", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER BV, NL, vol. 125, no. 2, 27 July 2007 (2007-07-27) , pages 489-497, XP022296106, ISSN: 0925-4005, DOI: 10.1016/J.SNB.2007.02.058

**Description**

Le domaine de l'invention est celui de l'identification objective d'ensemble de molécules volatiles pouvant correspondre à une odeur ou à un arôme.

Un réel besoin est apparu, notamment dans les moyens pouvant être développés pour retranscrire l'authenticité ou la traçabilité dans le domaine des marques olfactives, du développement d'emballages ou encore dans le domaine de l'alimentaire.

En effet, il peut être particulièrement intéressant de relier une couleur ou un ensemble de couleurs à une odeur ou à un arôme, les données visuelles étant moins complexes et de reconnaissance plus facile. Ce type de corrélation peut trouver des applications dans tous les domaines d'activité dans lesquels il existe un besoin de transcrire, de manière objective un mélange odorant en un ensemble de paramètres objectifs, notamment en des données colorimétriques objectives et inversement.

Actuellement cette attribution est réalisée de manière humaine comme par exemple dans l'article Yu-Jin Kim « Can eyes smell ? Cross modal correspondence between color hue-tone and frangrance family » Color Research & Application vol 38 num 2, 31 oct 2011, pages 139-156. On demande à des juges experts d'évaluation sensorielle de réaliser les profils colorés d'odeur c'est-à-dire d'attribuer une couleur ou une pluralité de couleurs à une odeur.

Cette solution n'est pas satisfaisante du fait de la difficulté de formation des juges et du caractère chronophage et coûteux de ce type de mesure. De plus, du fait de la valence hédonique des odeurs, ce type de procédé de reconnaissance, nécessite beaucoup de tests, le panel de juges doit avoir une taille très importante pour limiter la variabilité et obtenir des résultats fiables. Cette solution ne permet pas de décrire précisément une odeur au moyen d'un ensemble de couleurs ni d'attribuer une couleur, voir un panel de couleurs à une nouvelle odeur qui n'aurait pas été préalablement testée par les juges. Autrement dit, on ne peut pas attribuer une profil coloré à une nouvelle odeur sans que celui-ci ne soit réalisé par un panel de juges, ce qui, comme nous l'avons dit précédemment est consommateur de temps du fait de la nécessité de former les juges et de faire intervenir un panel suffisamment important de juges pour obtenir un profil chromatique le plus objectif possible. Enfin, il n'est pas possible, via cette méthode de réaliser l'étape inverse qui est d'attribuer une odeur ou un arôme à un panel de couleurs.

En outre le document US 2009/0271003 décrit un système intégré d'éclairage et de dispersion de parfums employant un réseau de neurones artificiels afin de transcrire un parfum à disperser sélectionné par un utilisateur en une couleur d'éclairage à diffuser conjointement et vice-versa.

Le problème que se propose de résoudre l'invention est de proposer un procédé permettant d'attribuer, de manière objective un profil chromatique à un mélange odorant quelconque (et inversement) permettant de s'affranchir de tout ou partie des inconvénients listés précédemment.

A cet effet, l'invention a pour objet un procédé de transcription d'une odeur ou d'un arôme en une description colorimétrique comprenant les étapes suivantes :

- une première étape d'analyse physico-chimique de ladite odeur ou dudit arôme afin de lui associer une description physico-chimique comprenant un vecteur physico-chimique, comprenant des proportions associées à un ensemble prédéterminé de molécules volatiles respectives,
- une deuxième étape d'analyse physico-chimique d'un ensemble d'odeurs et/ou d'arômes dits de test afin d'attribuer à chaque odeur ou arôme test dudit ensemble, une description physico-chimique dite de test comprenant un vecteur physico-chimique dit de test comprenant des proportions test associées audit ensemble prédéterminé de molécules volatiles respectives,
- une étape d'attribution, aux odeurs et/ou arômes test de descriptions colorimétriques test respectives comprenant des proportions dites test associées à un ensemble de couleurs respectives,
- une étape d'attribution, à l'odeur ou à l'arôme, d'une description colorimétrique comprenant des proportions associées auxdites couleurs respectives, ladite étape d'attribution comprenant une première étape d'attribution, à l'odeur ou à l'arôme, d'une description colorimétrique théorique comprenant des proportions colorimétriques théoriques associées auxdites couleurs respectives, cette première étape d'attribution étant réalisée au moyen d'un premier réseau de neurones artificiels présentant une architecture en couches, en lui présentant, en entrée le vecteur physico-chimique, ledit premier réseau de neurones générant en sortie, ladite description colorimétrique théorique, ledit premier réseau de neurones étant préalablement soumis à une première étape d'apprentissage lors de laquelle on lui fournit, en entrée, des vecteurs physico-chimiques associés à l'ensemble d'odeurs et/ou d'arômes test, ladite première étape d'apprentissage étant réalisée de manière supervisée en tenant compte des descriptions colorimétriques test associées à l'ensemble d'odeurs et/ou d'arômes test respectifs.

Avantageusement, le procédé comprend, préalablement à la première étape d'apprentissage une étape de fixation lors de laquelle on fixe des poids de premières liaisons synaptiques reliant deux à deux des neurones dudit réseau de neurones de façon que les poids associées aux premières liaisons synaptiques ne soient pas modifiables lors de l'étape d'apprentissage.

Avantageusement, l'étape de fixation est réalisée à partir de premiers coefficients de corrélation, chaque premier coefficient de corrélation étant calculé entre une molécule de l'ensemble de molécules et une couleur de l'ensemble de couleurs, calculés dans une étape préalable à partir de vecteurs physico-chimiques test et des descriptions colorimétriques associées aux odeurs et/ou arômes test (auxquels lesdits vecteurs physico-chimiques sont attribués.

Avantageusement, l'étape préalable comprend, pour au moins une couleur d'indice j :

- une étape de sélection, dans l'ensemble d'odeurs et/ou arômes test, d'un sous-ensemble d'indice j d'odeurs et/ou d'arômes test qui sont associés à des descriptions colorimétriques test présentant des proportions non nulles pour ladite couleur d'indice j,
- une première étape de regroupement des odeurs et/ou arômes test du sous ensemble d'indice j dans un ensemble de groupes d'odeurs et/ou d'arômes test, de façon que les vecteurs physico-chimiques associés aux odeurs et/ou arômes test présents dans un groupe présentent entre eux des premiers coefficients de corrélation plus importants qu'avec les vecteurs physico-chimiques associés aux odeurs et/ou arômes test présents dans les autres groupes dudit ensemble de groupes,
- et, pour au moins un desdits groupes,

    - une étape d'élaboration d'une liste de molécules communes correspondant aux molécules dont la proportion test est non nulle dans tous les vecteurs physico-chimiques test associés aux odeurs et/ou arômes test regroupés dans ledit groupe,
    - et, pour au moins une molécule prise dans la liste de molécules communes,

        - étape lors de laquelle on établit une première suite dans laquelle on ordonne les odeurs et/ou arômes test regroupés dans ledit groupe selon l'ordre croissant de la proportion de ladite molécule dans les vecteurs physico-chimiques test associés auxdites odeurs et/ou arômes test,
        - une étape lors de laquelle on établit une deuxième suite dans laquelle on ordonne les odeurs et/ou arômes test regroupés dans ledit groupe selon l'ordre croissant de la proportion de ladite couleur dans les descriptions colorimétriques associées auxdites odeurs et/ou arômes test,
        - une étape de calcul d'un autre coefficient de corrélation entre la première suite et la deuxième suite,

- une étape de calcul d'au moins un premier coefficient de corrélation entre une molécule de l'ensemble de molécules et une couleur de l'ensemble de couleurs à partir des autres coefficients de corrélation calculés pour ladite molécule et ladite couleur.

Cette caractéristique permet de faciliter l'apprentissage du réseau de neurones.

Selon un mode de réalisation, la description colorimétrique est la description colorimétrique théorique.

Selon un autre mode de réalisation, l'étape d'attribution de la description colorimétrique comprend une étape d'amélioration de la description colorimétrique théorique à partir de valeurs d'un ensemble de descripteurs sensoriels attribuées à l'odeur ou à l'arôme et à des odeurs et/ou arômes test respectifs de façon à obtenir ladite description colorimétrique, l'ensemble de descripteurs sensoriels comprenant au moins un descripteur sensoriel pris parmi une source d'une odeur ou d'un arôme, un indice de comestibilité, un indice d'agréabilité, un indice de familiarité, une note olfactive, un indice d'intensité.

Cette caractéristique permet d'affiner le profil chromatique théorique (description colorimétrique) en intégrant la valence hédonique de l'odeur ou de l'arôme.

Avantageusement, l'étape d'amélioration comprend :

- une étape de soumission d'au moins un deuxième réseau de neurones artificiels d'indice b à une deuxième étape d'apprentissage, un deuxième réseau de neurones présentant une architecture en couches comprenant une couche d'entrée apte à recevoir des valeurs associées à l'ensemble de descripteurs sensoriels et fournissant en sortie des première Lm, deuxième cm et troisièmes Hpm coordonnées moyennes, où p = est un entier allant de 1 à v entier, dans un repère colorimétrique dit L, C, H, ledit deuxième réseau de neurones recevant, lors de la deuxième étape d'apprentissage, des valeurs de l'ensemble de descripteurs sensoriels associées aux odeurs et/ou arômes test présents dans une série couleur constituée d'au moins une partie des odeurs et/ou arômes test de l'ensemble d'odeurs et/ou arômes test,
- une deuxième étape d'analyse (de l'odeur ou de l'arôme en fournissant, les valeurs de l'ensemble de descripteurs sensoriels en entrée dudit deuxième réseau de neurone d'indice b qui fournit en sortie, des première Lmbutb, deuxième cmbutb et troisièmes Hpmbutb coordonnées moyennes cibles d'indice b dans le repère L, H, C, d'une description colorimétrique cible associée à l'odeur ou à l'arôme considéré,

- une étape de calcul, d'une nouvelle description colorimétrique comprenant de nouvelles proportions qcnb$_j$ qui respectent, quelque soit j compris entre 1 et J, les équations suivantes :

$$Lmbutb = \sum_{j=1}^{J} Lj * qcnb_j$$

$$cmbutb = \sum_{j=1}^{J} cj * qcnb_j \ ,$$

$Hpmbutb = \sum_{j=1}^{J} PHpj * qcnb_j$ avec p entier allant de 1 à v et v entier, où Lj, cj et PHpj sont les coordonnées respectives de la couleur Cj d'indice j dans le repère L, H, C.

et qui minimisent, pour au moins une couleur Cj, la valeur absolue |qcnb$_j$-qcTb$_j$| de la différence entre la nouvelle proportion d'ordre b et la proportion théorique d'ordre b.

Avantageusement, le procédé comprend, préalablement à l'étape d'amélioration, une deuxième étape de regroupement d'odeurs et/ou d'arômes test appartenant à l'ensemble des odeurs et/ou arômes test dans un ensemble de séries dites séries couleurs, par similarité des descriptions colorimétriques test qui leurs sont attribuées.

Avantageusement, le procédé comprend :

- une étape de calcul de coefficients de similarité entre l'odeur et les séries couleurs respectives, à partir des valeurs de l'ensemble de descripteurs sensoriels attribuées à l'odeur ou à l'arôme et aux odeurs et/ou arômes test regroupés dans des séries couleurs respectives et/ou à partir de la description colorimétrique théorique et à partir des descriptions colorimétriques test attribués aux odeurs test regroupées dans les séries d'odeurs respectives,
- une étape de vérification lors de laquelle on identifie, parmi les séries couleurs, des séries couleurs similaires avec lesquelles l'odeur ou l'arôme vérifie un premier critère de similarité prédéterminé, et lors de laquelle on vérifie si l'odeur ou l'arôme vérifie un deuxième critère de similarité avec une série couleur prise parmi les séries couleurs similaires,
- et, lorsque l'odeur ou l'arôme vérifie le premier et le deuxième critère de similarité, on soumet, lors de la deuxième étape d'analyse les valeurs de l'ensemble de descripteurs sensoriels attribués à l'odeur ou à l'arôme à un unique deuxième réseau de neurones préalablement soumis à l'étape d'apprentissage lors de laquelle on lui fournit en entrée les valeurs de l'ensemble de descripteurs sensoriels attribuées aux odeurs et/ou arômes test regroupés dans ladite série couleur avec laquelle l'odeur ou l'arôme vérifie le deuxième critère de similarité,
- et, lorsque l'odeur ou l'arôme vérifie le premier critère de similarité mais pas le deuxième critère de similarité, on soumet, lors de la deuxième étape d'analyse, les valeurs de l'ensemble de descripteurs sensoriels attribués à l'odeur ou à l'arome à une pluralité de deuxième réseau de neurones préalablement soumis à l'étape d'apprentissage lors de laquelle on lui fournit en entrée les valeurs de l'ensemble de descripteurs sensoriels attribuées aux odeurs et/ou arômes test regroupés dans les séries couleur avec laquelle l'odeur ou l'arôme vérifie le premier critère de similarité, les proportions colorimétriques associées auxdites couleurs respectives étant des combinaisons linéaires des nouvelles proportions colorimétriques associées aux couleurs respectives.

Avantageusement, le procédé comprend, préalablement à l'étape d'amélioration, une étape de correction consistant à mettre à jour la description colorimétrique théorique à partir des valeurs de l'ensemble de descripteurs associées à l'odeur ou à l'arôme et à partir d'une règle globale permettant de calculer, pour au moins une couleur, et à partir de valeurs de l'ensemble de descripteurs sensoriels attribués à l'arôme ou à l'odeur, une probabilité de présence, dans une description colorimétrique associée à ces valeurs, d'une proportion colorimétrique associée à ladite couleur, ladite étape de correction étant suivie d'un retour à l'étape de calcul de coefficients de similarité.

Avantageusement, l'étape d'attribution, aux odeurs et/ou arômes test, d'une description colorimétrique comprenant des proportions test associées à un ensemble de couleurs respectives, comprend :

- une étape d'attribution de couleurs dites couleurs jugées à des odeurs et/ou arômes de test (par un ensemble de juges humains,
- une étape d'élaboration d'une description colorimétrique dite de test de chaque odeur ou arôme test comprenant des proportions test associées à un ensemble de couleurs respectives à partir de la proportion de juges, qui parmi les juges appartenant à un autre ensemble de juges comprenant tout ou partir dudit ensemble de juges, ont attribué chacune des couleurs jugées à ladite odeur ou audit arôme de test.

Avantageusement, les proportions test relatives aux différentes couleurs sont déterminées à partir de la proportion de juges qui parmi les juges apparentant à l'autre ensemble de juges, ont attribué chacune des couleurs jugées audit arôme ou à ladite odeur et à partir de coefficients de proximité calculés entre la couleur jugée et les couleurs de l'ensemble de couleurs.

Avantageusement, le procédé comprend une étape de représentation de la nouvelle odeur ou du nouvel arôme au moyen d'une carte chromatique dans laquelle le pourcentage de la surface de la carte chromatique occupé par une couleur de l'ensemble de couleurs correspond au rapport entre proportion associée à ladite couleur dans la description colorimétrique et la somme des proportions associées à un sous-ensemble de l'ensemble de couleurs dans la description colorimétrique, le sous-ensemble étant choisi de manière à ce que la somme des proportions associées aux couleurs qu'il contient, dans la description colorimétrique, est au moins égale à un seuil prédéterminé.

L'invention a également pour objet un procédé de transcription d'une description colorimétrique initiale comprenant des proportions colorimétriques initiales relatives à un ensemble de couleurs respectives en une description physico-chimique de résultat comprenant une liste de molécules comprenant les étapes suivantes :

- une deuxième étape d'analyse physico-chimique d'un ensemble d'odeurs et/ou d'arômes dits de test afin d'attribuer à chaque odeur ou arôme test dudit ensemble, une description physico-chimique dite de test comprenant un vecteur physico-chimique dit de test comprenant des proportions test associées audit ensemble prédéterminé de molécules volatiles respectives,
- une première étape d'attribution, aux odeurs et/ou arômes test de descriptions colorimétriques test respectives comprenant des proportions dites test associées à un ensemble de couleurs respectives,
- une deuxième étape d'attribution, d'une description colorimétrique hypothétique comprenant des proportions colorimétriques hypothétiques à l'ensemble de couleurs respectives, à un vecteur physico-chimique hypothétique comprenant des proportions hypothétiques associées audit ensemble de molécules volatiles respectives, cette deuxième étape d'attribution étant réalisée au moyen d'un premier réseau de neurones artificiels présentant une architecture en couches, en lui présentant, en entrée un vecteur physico-chimique hypothétique comprenant des proportions hypothétiques associées audit ensemble de molécules volatiles respectives, ledit premier réseau de neurones générant en sortie, ladite description colorimétrique hypothétique, ledit premier réseau de neurones étant préalablement soumis à une première étape d'apprentissage lors de laquelle on lui fournit, en entrée, des vecteurs physico-chimiques associés à l'ensemble d'odeurs et/ou d'arômes test, ladite première étape d'apprentissage étant réalisée de manière supervisée en tenant compte des descriptions colorimétriques test associées à l'ensemble d'odeurs et/ou d'arômes test respectifs,
- une étape de calcul d'erreur dans laquelle on calcule une erreur représentative d'un écart entre la description colorimétrique hypothétique et la description colorimétrique initiale, ladite étape de calcul d'erreur étant suivie d'une étape de mise à jour du vecteur physico-chimique hypothétique, à partir de l'erreur et du vecteur physico-chimique hypothétique, et de retour à l'étape d'attribution tant que l'erreur est supérieure à un seuil d'erreur prédéterminé, la description physico-chimique de résultat étant déterminée à partir du vecteur physico-chimique hypothétique qui engendre une erreur de calcul inférieure ou égale à l'erreur.

Avantageusement, la description initiale est associée à des valeurs, dites initiales, d'un ensemble de descripteurs sensoriels et les odeurs et/ou arômes test sont associés à des valeurs dudit ensemble de descripteurs sensoriels, ledit ensemble de descripteurs sensoriels comprend au moins un descripteur sensoriel pris parmi une source d'une odeur ou d'un arôme, un indice de comestibilité, un indice d'agréabilité, un indice de familiarité, une note olfactive, un indice d'intensité, et comprenant une étape préalable de détermination du vecteur physico-chimique hypothétique à partir des valeurs dites initiales de l'ensemble de descripteurs sensoriels et à partir des valeurs dudit ensemble de descripteurs sensoriels associés à l'odeur ou à l'arôme test.

Avantageusement, le procédé comprend, préalablement à l'étape de détermination du vecteur physico-chimique hypothétique :

- une étape de construction d'une autre loi globale permettant de déterminer, à partir des valeurs d'un ensemble de descripteurs sensoriels associées à une odeur ou un arôme , les probabilités de présence de l'ensemble de molécules respectives dans l'odeur ou de l'arôme O soient non nulles, cette étape étant réalisée à partir des vecteurs physico-chimiques test et des valeurs de l'ensemble de descripteurs sensoriels associés aux odeurs et/ou arômes test,
- une étape de regroupement des odeurs et/ou arômes test dans un ensemble de séries couleurs par similarité des leurs descriptions colorimétriques test respectives,

L'étape de détermination du vecteur physico-chimique hypothétique comprenant :

- une étape de détermination de probabilités de présence, dans l'odeur ou l'arôme résultat, de l'ensemble des molécules respectives, cette étape étant réalisée à partir des valeurs initiales de l'ensemble de descripteurs sensoriels et à partir de l'autre loi globale,
- une étape d'établissement d'une liste de molécules possibles prises dans l'ensemble de molécules, cette liste de molécules possibles correspondant aux molécules qui, parmi les molécules de l'ensemble de molécules, présentent une probabilité de présence non nulle,
- une étape de calcul de coefficients de corrélation entre la description initiale et les séries couleurs respectives à partir des valeurs initiales de l'ensemble de descripteurs sensoriels et des valeurs de l'ensemble de descripteurs sensoriels associées aux odeurs et/ou arômes test respectifs ainsi qu'à partir des descriptions colorimétriques test et initiales,
- une étape de vérification lors de laquelle on identifie, parmi les séries couleurs, les séries couleurs avec lesquelles la description initiale vérifie un critère de corrélation prédéterminé, cette étape est réalisée à partir des coefficients de similarité cités au paragraphe précédent,
- une étape d'élaboration du vecteur physico-chimique hypothétique à partir de la liste de molécules possibles et à partir des vecteurs physico-chimiques test associés aux odeurs et/ou arômes test compris dans les séries couleurs avec lesquelles la description initiale vérifie le critère de corrélation, le vecteur physico-chimique hypothétique comprenant des proportions hypothétiques associées aux molécules Mi possibles respectives.

D'autres caractéristiques et avantages de l'invention apparaîtront à la lecture de la description détaillée qui suit, faite à titre d'exemple non limitatif et en référence aux dessins annexés dans lesquels :

- la figure 1a représente schématiquement les étapes d'un exemple de procédé de transcription d'une odeur ou d'un arôme en une description colorimétrique selon l'invention et la figure 1b représente plus précisément l'étape d'attribution d'une description colorimétrique à l'odeur ou à l'arôme,
- la figure 2 représente plus en détail un exemple de réalisation de l'étape d'attribution de descriptions colorimétriques test à l'ensemble des odeurs et/ou arômes test,
- la figure 3 représente schématiquement un premier réseau de neurones utilisé dans le procédé selon l'invention,
- la figure 4 représente schématiquement un exemple de réalisation de l'étape préalable de calcul des premiers coefficients de corrélation,
- la figure 5 représente schématiquement un exemple de représentation d'une description colorimétrique,
- la figure 6 représente schématiquement un autre exemple de description colorimétrique,

D'une figure à l'autre, les mêmes éléments sont repérés par les mêmes références.

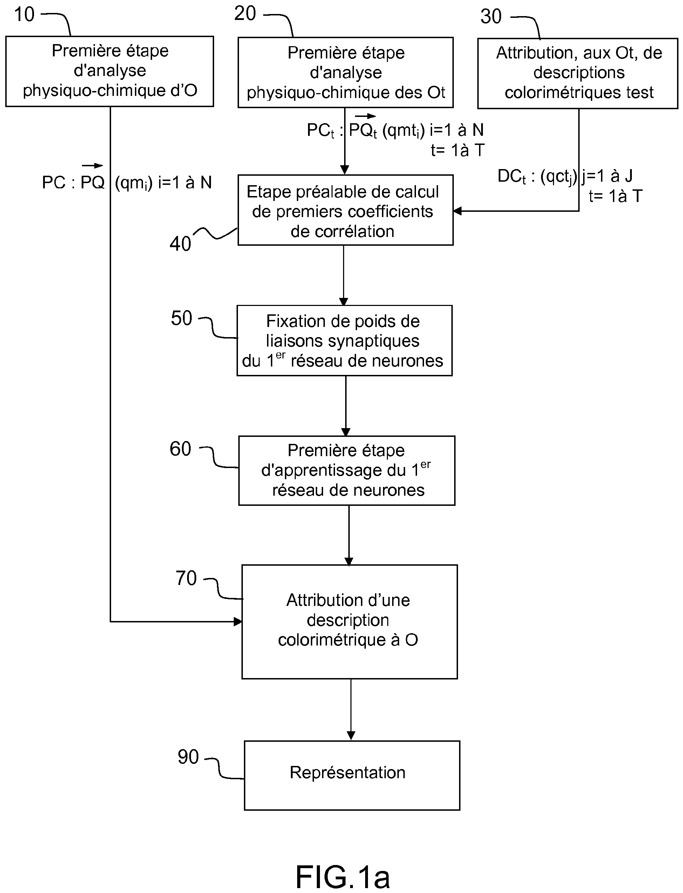

Sur la figure 1a, on a représenté les étapes du procédé selon l'invention de transcription d'une odeur ou d'un arôme O en une description colorimétrique aussi appelée profil chromatique. Une odeur ou un arôme est un ensemble de molécules volatiles aptes à être perçues de manière olfactive directement, c'est-à-dire par le nez, pour une odeur, ou indirectement, c'est-à-dire par la bouche, pour un arôme.

Ce procédé comprend une étape 10 d'analyse physico-chimique de l'odeur ou de l'arôme O afin de lui associer une description physico-chimique, appelée description physico-chimique PC comprenant un vecteur physico-chimique PQ comprenant une liste de proportions $qm_i$ relatives à un ensemble prédéterminé de molécules volatiles respectives Mi avec i = 1 à N où N est le nombre de molécules volatiles compris dans un ensemble prédéterminé de molécules. La proportion d'une molécule dans le mélange correspond à sa proportion relative dans ce mélange.

Actuellement, le nombre N de molécules est de 458 mais ce nombre peut évoluer.

La somme des proportions comprises dans chaque vecteur correspond à 1.

Cette étape 10 d'analyse physico-chimique est une étape connue de l'homme du métier. Elle est, par exemple, réalisée au moyen d'un nez électronique qui est apte à capter les molécules volatiles formant une odeur ou un arôme et à les identifier ainsi que la proportion des molécules qui sont comprises dans le mélange de molécules volatiles. Le nez électronique réalise le chromatogramme de l'odeur ou de l'arôme et identifie, à partir du chromatogramme obtenu, les molécules présentes et leurs concentrations (ou proportions) respectives dans le mélange de molécules volatiles à l'origine de l'odeur ou de l'arôme.

Un exemple de méthode de ce type est décrit dans la demande de brevet EP1566633.

La description physico-chimique peut également comprendre un vecteur physico-chimique dit de pertinence PCP comprenant des indices de pertinence IPi associés à l'ensemble des molécules respectives Mi. Un indice de pertinence correspond à la probabilité que la molécule soit effectivement présente dans le mélange olfactif ou aromatique.

Ce procédé comprend également une étape 20 d'analyse physico-chimique d'un ensemble d'odeurs et/ou d'arômes Ot dits de test d'indices t (avec t= 1 à T où T est le nombre d'arômes et/ou odeurs test) afin d'attribuer à chaque odeur ou arôme test Ot d'indice t une description physico-chimique dite de test $PC_t$ d'indice t comprenant un vecteur physico-chimique test $PQ_t$ comprenant une liste de proportions test $qmt_i$ associées audit ensemble prédéterminé de molécules respectives Mi. Ces descriptions physico-chimiques constituent une première base de données appelée base de données physico-chimique. La somme des proportions comprises dans un vecteur physico-chimique test est égale à 1.

La description physico-chimique test peut également comprendre un vecteur physico-chimique test de pertinence $PCP_t$ comprenant des indices de pertinence test $IPt_i$ associés à l'ensemble des molécules respectives Mi. Un indice de pertinence correspond à la probabilité que la molécule soit effectivement présente dans le mélange olfactif ou aromatique de test.

Si, lors de l'étape d'analyse physico-chimique, on trouve une nouvelle molécule qui n'est pas comprise dans l'ensemble de molécules Mi (i = 1 à I), elle peut mettre à jour l'ensemble de molécules de façon à ce qu'il intègre cette nouvelle molécule. Les vecteurs physico-chimiques existants sont alors mis à jour de façon à y intégrer des proportions liées à cette nouvelle molécule, proportions qui sont nulles pour les vecteurs existants. Il en est de même pour les vecteurs physico-chimiques de pertinence.

Le procédé comprend également une étape 30 d'attribution, aux odeurs et/ou arômes test Ot test d'indice t, c'est-à-dire aux vecteurs physico-chimiques test de quantité PQt d'indice t, de descriptions test DCt comprenant des proportions colorimétriques test $qct_j$ d'indice t associées à un ensemble de couleurs respectives Cj avec j= 1 à J où J est le nombre de couleurs dans l'ensemble de couleurs.

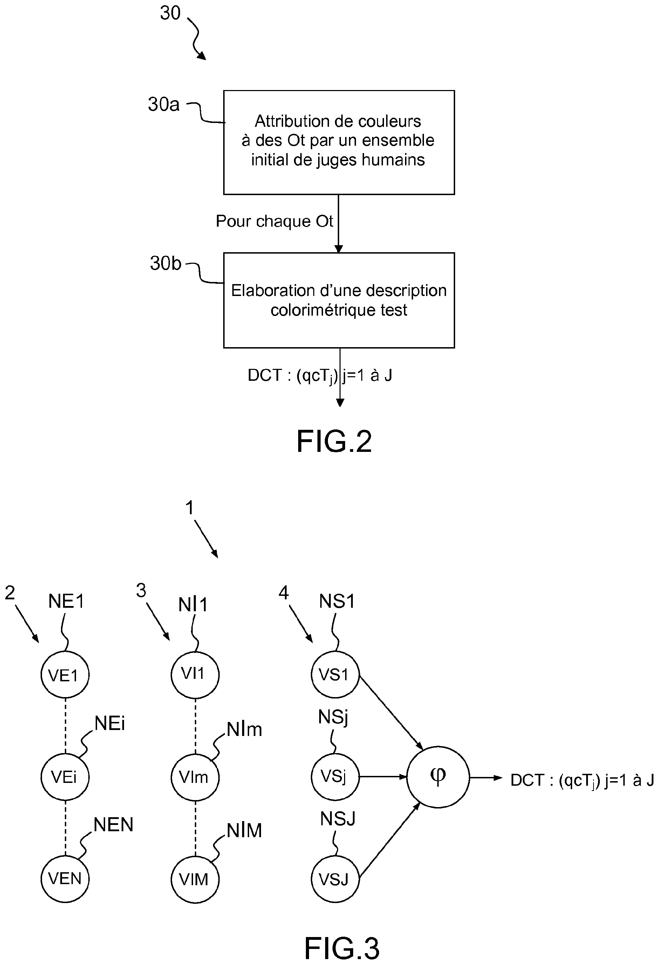

Cette étape 30 d'attribution est réalisée en partie par des juges humains. Elle comprend des étapes qui sont représentées sur la figure 2.

Chaque odeur ou arôme test Ot est présentée à un ensemble de juges humains. Chaque juge attribue une couleur dite couleur jugée, caractérisée par ses coordonnées dans un repère tridimensionnel LCH : L (luminosité), C (saturation), H (teinte) (étape 30a).

A partir de ces résultats, on élabore une description colorimétrique DCt de chaque odeur ou arôme test Ot, par exemple, sous forme d'un vecteur couleur test $CT_t$, comprenant des proportions colorimétriques test $qct_j$ associées à un ensemble de couleurs respectives $C_j$ à partir de la proportion de juges, qui parmi les juges d'un autre ensemble de juges, constitué de tout ou partie de juges de l'ensemble de juges, ont attribué chacune des couleurs jugées à ladite odeur ou audit arôme de test (étape 30b).

On peut permettre aux juges de choisir dans un ensemble discret ou continu de couleurs jugées.

Dans un mode de réalisation, l'ensemble de couleurs correspond à l'ensemble des couleurs jugées ayant été attribuées par des juges aux odeurs et arôme test. La proportion attribuée à une couleur, pour une odeur ou un arôme test, de l'ensemble de couleur est alors égale à la proportion de juges ayant attribué ladite couleur à l'odeur ou à l'arôme test.

En variante, l'ensemble de couleurs est un ensemble prédéterminé de couleurs espacées dans le repère tridimensionnel pouvant être différentes de l'ensemble des couleurs jugées.

Dans ce cas, les proportions colorimétriques test $qct_j$ relatives aux différentes couleurs $C_j$ sont déterminées à partir de la proportion de juges ayant attribué chacune des couleurs jugées audit arôme ou à ladite odeur test et à partir de coefficients de proximité calculés entre la couleur jugée et les couleurs de l'ensemble de couleurs.

L'autre ensemble de juges peut être l'ensemble des juges.

En variante, l'autre ensemble comprend seulement une partie des juges de l'ensemble de juges. Dans ce cas, le procédé comprend avantageusement une étape, non représentée de filtrage de l'ensemble de juges en fonction d'au moins une caractéristique des juges prise parmi leur âge, leur nationalité, leur genre, afin de construire l'autre ensemble de juges. En effet, les caractéristiques comme l'âge, la nationalité ou le genre des juges peuvent avoir un impact sur les couleurs qu'ils affectent aux odeurs et/ou arômes test. Ce filtrage permet de cibler beaucoup plus précisément le profil chromatique de façon à répondre à différentes demandes marketing. On peut par exemple cibler un profil chromatique pour des cultures non-européennes.

Avantageusement, l'ensemble de juges comprend un nombre de juge au moins égal à 100. Cela permet d'obtenir au final une description colorimétrique la plus objective et reproductible possible.

Ce procédé comprend également une étape d'attribution 70 à l'odeur ou à l'arôme O d'une description colorimétrique DC comprenant des proportions colorimétriques $qc_j$ associées à des couleurs $C_j$ respectives de l'ensemble de couleurs comprenant J couleurs (j = 1 à J). La somme des proportions associée aux couleurs est égale à 1.

Cette étape 70 comprend une première étape d'attribution 71 (à l'odeur ou à l'arôme O, d'une description colorimétrique théorique DCT comprenant des proportions colorimétriques théoriques $qcT_j$ associées aux couleurs Cj respectives de l'ensemble de couleurs comprenant J couleurs (j = 1 à J). La somme des proportions associée aux couleurs est égale à 1.

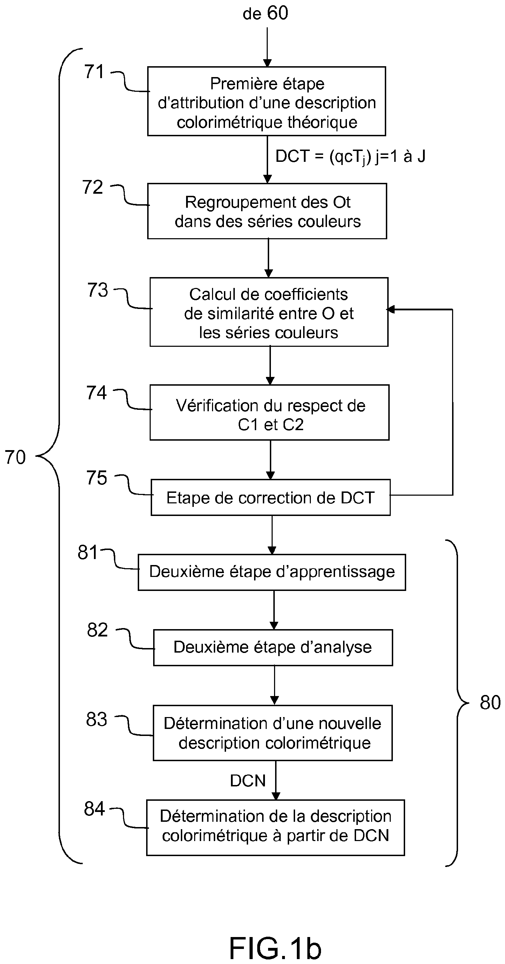

L'étape 71, représentée sur la figure 1b, est réalisée au moyen d'un premier réseau de neurones artificiels 1 du type perceptron multicouches dont un exemple est représenté sur la figure 3. Ce réseau de neurones 1 présentent une architecture en couches comprenant des couches successives comprenant une couche d'entrée 2 présentant une pluralité de neurones d'entrée NEi (i= 1 à N) dont le nombre est égal au nombre de molécules Mi et fournissant en sortie, des valeurs VEi, appelées valeurs des neurones d'entrée respectifs qui sont égales aux proportions $qc_j$ associées aux molécules Mi respectives d'un vecteur physico-chimique PQ qui lui est fourni en entrée.

Le réseau de neurones artificiel 1 comprend en outre au moins une couche intermédiaire 3 comprenant une pluralité de neurones intermédiaires NIm (m= 1 à M et M est le nombre de neurones de la couche intermédiaire) fournissant en sortie des valeurs VIm, appelées valeurs des neurones intermédiaires respectifs. Dans l'exemple de la figure 3, le réseau de neurones comprend une unique couche intermédiaire.

Le réseau de neurones 1 comprend en outre une couche de sortie 4 comprenant une pluralité de neurones de sortie NSj, (j= 1 à J) dont le nombre est égal au nombre de couleurs de l'ensemble de couleurs défini précédemment et fournissant en sortie des valeurs respectives VSj, appelées valeurs des neurones de sortie respectifs NSj, qui sont associées aux couleurs respectives Cj.

Chaque neurone d'une couche est relié, au moyen de liaisons synaptiques, à tous les neurones de la couche précédente, lorsqu'elle existe, et à tous les neurones de la couche suivante, lorsqu'elle existe. Des poids synaptiques sont attribués à chaque liaison synaptique. Une liaison synaptique relie entre eux deux neurones. Autrement dit, dans l'exemple de la figure 3, le premier réseau de neurones comprend des liaisons synaptiques reliant chaque neurone d'entrée à chaque neurone intermédiaire et chaque neurone intermédiaire à chaque neurone de sortie. Pour plus de clarté, ces liaisons synaptiques ne sont pas représentées sur la figure 3.

La valeur VIm d'un neurone intermédiaire NIm est égale à une somme pondérée des valeurs des neurones de la couche précédente, dans laquelle la valeur de chaque neurone de la couche précédente est pondérée par les poids de la liaison synaptique reliant le neurone de la couche précédente et le neurone intermédiaire NIm considéré.

La valeur d'un neurone de sortie est égale à la somme pondérée des valeurs, dites valeurs d'activation, obtenues en appliquant une fonction d'activation fa aux valeurs respectives des neurones de la couche précédente, chaque valeur d'activation étant pondérée par le poids de la liaison synaptique reliant le neurone de sortie considéré et le neurone de la couche précédente considéré.

La fonction d'activation fa est, par exemple, la suivante :

$$\text{fa}(\text{VIm}) = \frac{1}{1+e^{-\text{VIm}}}$$

quelque soit m entier compris entre 1 et M

Nous allons décrire plus précisément, à titre non limitatif, les valeurs des neurones du premier réseau de neurones dans le cas, représenté sur la figure 3, où le premier réseau ne comprend qu'une seule couche intermédiaire comprenant un nombre M de neurones intermédiaires égal au nombre J de couleurs de l'ensemble de couleurs.

La valeur de sortie VIm d'un neurone intermédiaire NIm est, quelque soit m entier compris entre 1 et M, égal à : $VIm = \sum_{i=1}^{N} P_{i,m} * VE_i$ où $P_{i,m}$ est le poids de la liaison synaptique reliant le neurone NEi et le neurone NIm et où $VE_i$ est la valeur du neurone NEi.

La valeur VSj d'un neurone de sortie est égale à : $VSj = \sum_{m=1}^{J} K_{m,j} * fa(VIm)$ où $K_{m,j}$ est le poids de la liaison synaptique reliant le neurone de sortie NSj et le neurone intermédiaire NIm.

Les valeurs VSj sont comprises dans l'intervalle ]-∞ ; +∞[. Or, les proportions colorimétriques qc$_j$ associées aux couleurs Cj sont comprises entre 0 et 1. On applique donc une fonction non linéaire $\varphi(VSj)$ aux valeurs des neurones de sortie de façon à obtenir les proportions qc$_j$.

On peut, par exemple, mais de façon non limitative, utiliser la fonction suivante :

$$\varphi(VSj) = \frac{t_j}{\Sigma_{j=1}^{J} t_j} \text{ où } t_j = VSj \quad si\ VSj > 0\ et\ O\ si\ VSj \leq 0$$

Pour déterminer les poids des liaisons synaptiques du premier réseau de neurones 1, les fonctions d'activation étant fixées, le premier réseau de neurones est soumis, préalablement à l'étape 70, à une première étape d'apprentissage 60.

La première étape d'apprentissage 60 est réalisée de manière supervisée en fournissant, en entrée dudit premier réseau de neurones 1 les vecteurs physico-chimiques test PQt respectifs et en tenant compte des descriptions colorimétriques DCt test associées aux odeurs et/ou arômes test Ot auxquels sont attribués les vecteurs physico-chimiques test respectifs.

Autrement dit, la première étape d'apprentissage est réalisée de telle façon que lorsque l'on fournit un vecteur physico-chimique test PQt en entrée dudit premier réseau, on obtienne en sortie de celui-ci une description colorimétrique la plus proche possible de la description colorimétrique test attribuée audit vecteur.

Une fois la phase d'apprentissage 60 réalisée, on procède à l'étape 70 d'attribution d'une description colorimétrique DC, à l'odeur ou à l'arôme O comprenant des proportions associées colorimétriques $qc_j$ aux couleurs Cj.

Cette étape d'attribution 70 comprend une première étape d'attribution 71, à l'odeur ou à l'arôme, d'une description colorimétrique théorique DCT comprenant une liste de proportions colorimétriques théoriques $qcT_j$ associées aux couleurs Cj respectives en fournissant le vecteur physico chimique PQ en entrée du premier réseau de neurones 1. La sortie du premier réseau de neurones 1 correspond à la description colorimétrique théorique DCT comprenant la liste des proportions théoriques $qcT_j$ associées aux couleurs Cj respectives.

Dans un premier mode de réalisation, la description colorimétrique DC est la description colorimétrique théorique DCT.

Avantageusement, mais non nécessairement, le procédé comprend, préalablement à la première étape d'apprentissage 60, une étape de fixation 50 lors de laquelle on fixe des poids de premières liaisons synaptiques reliant deux à deux certains neurones appelés faux neurones, de façon à ce que ces poids ne soient pas modifiables lors de l'étape d'apprentissage. Lors de la première étape d'apprentissage on fixe alors les poids des liaisons synaptiques autres que les premières liaisons synaptiques.

L'étape 50 est réalisée à partir de premiers coefficients de corrélation. Chaque premier coefficient de corrélation est calculé entre une molécule de l'ensemble de molécules et une couleur de l'ensemble de couleurs. Cette étape permet de limiter la durée de l'étape d'apprentissage du réseau de neurones.

Les premiers coefficients de corrélation sont calculés dans une étape préalable 40 à partir de vecteurs physico-chimiques test et des descriptions colorimétriques test associées à ces vecteurs physico-chimiques via les odeurs ou arômes test respectifs auxquels elles sont attribuées.

On décrit ci-dessous une manière de réaliser cette étape 40. Dans cette méthode on présuppose que ce sont des combinaisons de molécules qui se traduisent par des couleurs (ou des combinaisons de couleurs).

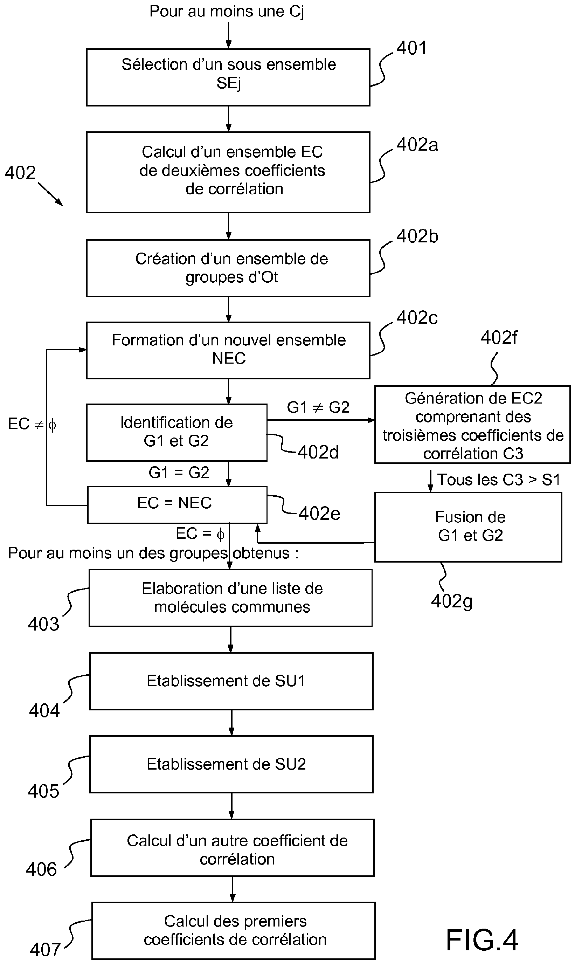

Cette étape 40 est détaillée sur la figure 4. Elle comprend pour, au moins une couleur Cj de l'ensemble de couleurs, une étape de sélection 401, dans l'ensemble d'odeurs et/ou arômes test Ot, d'un sous-ensemble SEj d'indice j d'odeurs et/ou d'arômes test qui sont associés à des descriptions colorimétriques test DCt (t= 1 à T) présentant des proportions colorimétriques test non nulles pour ladite couleur de même indice Cj.

L'idée est d'identifier différents groupes d'odeurs ou arômes qui présentent cette couleur dans leurs descriptions colorimétriques respectives. Par exemple, une odeur ou un arôme présentant une note acidulée ou lactée peut être associée à la couleur jaune. Or, ces notes acidulées et lactées proviennent de groupes de molécules complètement différentes. Autrement dit, différentes combinaisons de molécules peuvent renvoyer à la même couleur. On regroupe donc les odeurs et/ou arômes selon la similarité des molécules qui la composent.

Autrement dit, l'étape 40 comprend une étape de regroupement 402 des odeurs et/ou arômes test du sous ensemble SEj dans un ensemble de groupes d'odeurs et/ou d'arômes test, de façon que les vecteurs physico-chimiques PQt des odeurs et/ou arômes test présents dans un groupe présentent entre eux des premiers coefficients de corrélation plus importants qu'avec les vecteurs physico-chimiques PQt des odeurs et/ou arômes test présents dans les autres groupes dudit ensemble de groupes. Dans la suite du texte, par regroupement d'odeurs et/ou d'arômes test, dans des groupes ou séries, on entend qu'on regroupe, dans ces différents groupes ou séries, les vecteurs physico-chimiques test et/ou les valeurs d'un ensemble de descripteurs sensoriels test et/ou les descriptions physico-chimiques test qui sont associés à ces odeurs et/ou arômes test.

On a représenté, sur la figure 4, un exemple non limitatif d'étapes d'un procédé de regroupement 402 des odeurs et/ou arômes test présents dans le sous-ensemble SEj comprenant un nombre H d'odeurs et/ou d'arômes test.

Ce procédé comprend :

- une étape 402a de calcul de deuxièmes coefficients de corrélation entre chacun des vecteurs physico-chimiques associés aux odeurs et/ou arômes test compris dans le sous-ensemble SEj et les vecteurs physico-chimiques associés aux autres odeurs et/ou arômes test présents dans le sous-ensemble SEj de façon à obtenir un ensemble de coefficients de corrélation EC comprenant les deuxièmes coefficients de corrélation ,
- une étape 402b de création d'un premier ensemble de groupes comprenant un nombre de groupes égal au nombre H d'odeurs et/ou d'arômes test compris dans le sous-ensemble SEj, chaque groupe comprenant une odeur et/ou un arôme dudit sous-ensemble,
- une étape 402c de formation d'un nouvel ensemble de coefficients de corrélation NEC, à partir de l'ensemble de coefficients corrélation EC, le nouvel ensemble NEC correspondant à l'ensemble de coefficients de corrélation EC dans lequel on a supprimé le deuxième coefficient de corrélation le plus important qu'il contient,
- une étape d'identification 402d, parmi lesdits groupes de premier G1 et deuxième G2 groupes comprenant les deux odeurs et/ou arômes test Ot entre lesquels le deuxième coefficient de corrélation correspond au deuxième coefficient de corrélation le plus important contenu dans l'ensemble de corrélation EC,
- si les premier G1 et deuxième G2 groupes comprennent exactement les mêmes arômes et/ou odeurs test, le procédé comprend une étape 402e de mise à jour de l'ensemble de coefficients de corrélation EC, ce dernier étant remplacé par le nouvel ensemble de coefficients de corrélation NEC et on retourne à l'étape de formation 402c d'un nouvel ensemble de coefficients de corrélation NEC tant que l'ensemble de corrélation EC comprend au moins un deuxième coefficient de corrélation,

- sinon, on génère 402f un deuxième ensemble de coefficients de corrélation EC2 comprenant des troisièmes coefficients de corrélation C3 obtenus en corrélant chaque vecteur physico-chimique associé à une odeur ou arôme test présent dans le premier groupe G1 avec chaque vecteur physico-chimique associé à une odeur du arôme test présent dans le deuxième groupe G2, et si tous les troisièmes coefficients de corrélation sont supérieurs à un premier seuil S1 prédéterminé (égal, par exemple mais de façon non limitative à 0, 3) le procédé comprend une étape de fusion 402h entre les premier G1 et deuxième G2 groupes, dans laquelle on remplace, dans le premier ensemble de groupes, les premier et deuxième groupes par un unique groupe comprenant les odeurs et/ou arômes test présents dans ces deux groupes,

- puis on retourne à l'étape 402e.

A la fin de l'étape de regroupement 402, on a regroupé, les odeurs et/ou arômes test du sous-ensemble SEj en fonction de la similarité des vecteurs physico-chimiques respectifs.

Pour au moins un des groupes obtenus, on élabore 403 une liste de molécules communes correspondant aux molécules associées à une proportion test non nulle dans tous les vecteurs physico-chimiques test associés aux odeurs et/ ou arômes test regroupés dans ledit groupe et, pour au moins une molécule prise dans la liste des molécules communes :

- on établit 404 une première suite SU1 dans laquelle on ordonne les odeurs et/ou arômes test regroupés dans ledit groupe selon l'ordre croissant de la proportion de ladite molécule dans les vecteurs physico-chimiques associés auxdites odeurs et/ou arômes test,
- on établit 405 une deuxième suite SU2 dans laquelle on ordonne les odeurs et/ou arômes test regroupés dans ledit groupe selon l'ordre croissant de la proportion de ladite couleur dans les descriptions colorimétriques associées auxdites odeurs et/ou arômes test,
- on calcule 406 un autre coefficient de corrélation entre la première et la deuxième suite.

Autrement dit, dans la suite SU1, les proportions test associées à la molécule considéré dans les vecteurs physico-chimiques respectifs associés aux odeurs et/ou arômes test formant ladite suite, augmentent avec le numéro d'ordre des odeurs et/ou arômes test dans ladite suite. Dans la suite SU2, les proportions colorimétriques test associées à la molécule considérée dans les descriptions physico-chimiques associées aux odeurs et/ou arômes test respectives formant ladite suite, augmentent avec le numéro d'ordre des odeurs et/ou arômes test dans ladite suite.

La corrélation entre les deux suites SU1, SU2 exprime la probabilité que la molécule ait une relation statistique avec la couleur considérée, et plus précisément que l'augmentation, ou la diminution, de la proportion de la dite molécule dans un vecteur physico-chimique induise une augmentation, ou respectivement, une diminution de la proportion de la couleur dans les descriptions colorimétriques. Une corrélation égale à 1 signifie que l'augmentation de la proportion de la molécule correspond toujours à une augmentation du pourcentage de juges ayant sélectionné la couleur (c'est-à-dire de la proportion de la couleur), et une corrélation nulle signifie le contraire.

On calcule ensuite 407 le premier coefficient de corrélation entre une molécule de l'ensemble de molécules et une couleur de l'ensemble de couleurs à partir des autres coefficients de corrélation calculés pour ladite molécule et ladite couleur. Il s'agit, par exemple, d'une combinaison linéaire des coefficients des autres coefficients de corrélation.

Avantageusement, mais non nécessairement, l'étape 70 d'attribution de la description colorimétrique à l'odeur considérée comprend une étape d'amélioration 80 de la description colorimétrique théorique à partir de valeurs d'un ensemble de descripteurs sensoriels attribuées à l'odeur ou à l'arôme et aux odeurs et/ou aux arômes test Ot respectifs.

L'ensemble de descripteurs sensoriels comprend au moins un descripteur pris parmi la source de l'odeur ou de l'arôme considéré, un indice de comestibilité, un indice d'agréabilité, sa familiarité, une note olfactive, un indice d'intensité (explique ce à quoi cela correspond). Les descripteurs sensoriels peuvent prendre plusieurs valeurs dans des ensembles prédéterminés de valeurs. Par exemple, les indices respectifs peuvent prendre une pluralité de valeurs parmi des ensembles respectifs d'indices.

Le procédé selon l'invention comprend avantageusement mais non nécessairement, préalablement à l'étape d'amélioration 80, une étape de regroupement 72 des odeurs et/ou arômes test dans un ensemble de séries Sb d'odeurs et/ou arômes test d'ordre b (b = 1 à B) par similarité des leurs descriptions colorimétriques test respectives. Ces séries Sb sont appelées séries couleurs d'ordre b dans la suite du texte.

Autrement dit, le procédé comprend une étape de regroupement 72 des odeurs et/ou arômes test dans des séries couleurs, de façon que les descriptions colorimétriques test des odeurs et/ou arômes test présents dans une première série couleur présentent entre elles des coefficients de corrélation plus importants qu'avec les descriptions colorimétriques test des odeurs et/ou arômes test présents dans les autres séries couleur dudit ensemble de séries couleurs.

On peut regrouper, via l'étape 72, les odeurs et/ou arômes test en séries en utilisant le procédé 402 décrit précédemment utilisé pour regrouper des odeurs et/ou arômes test selon des groupes. Toutefois, dans ce procédé on calcule les deuxièmes, troisièmes et autres coefficients de corrélation, non plus entre les vecteurs physico-chimiques mais entre les descriptions colorimétriques. Par ailleurs, on part de l'ensemble des odeurs et/ou arômes test et non d'un sous-ensemble.

A la fin de ce procédé de regroupement, on a regroupé des odeurs et/ou arômes test par similarité des descriptions colorimétriques auxquelles ils sont respectivement associés.

L'étape d'amélioration 80 comprend également avantageusement une étape de soumission 81 d'au moins un deuxième réseau de neurones artificiel d'indice b à une étape d'apprentissage consistant à fixer des poids synaptiques des liaisons entre des neurones de couches adjacentes dudit deuxième réseau de neurones d'indice b.

Dans cette étape 81, un deuxième réseau de neurones d'indice b présente une architecture en couches comprenant une couche d'entrée apte à recevoir des valeurs de l'ensemble de descripteurs sensoriels associées aux odeurs et/ou arômes test appartenant à une série d'odeurs Sg constituée d'au moins une partie des odeurs et/ou des arômes test.

Le deuxième réseau de neurones qui reçoit des valeurs d'un ensemble de descripteurs sensoriels associées à une odeur, ou un arôme fournit en sortie des première Lm, deuxième cm et troisièmes coordonnées moyennes Hpm (avec p = 1 à v entier généralement inférieure à J - 2 mais pouvant être supérieur), dans un repère L, C, H, d'une description colorimétrique de cette odeur ou de cet arôme. Les coordonnées moyennes associées à une description colorimétrique comprenant des proportions $qc_j$ associées à des couleurs Cj de l'ensemble de couleurs comprenant J couleurs (j= 1 à J) sont données par les formules suivantes : $Lm = \sum_{j=1}^{J} Lj * qc_j$ où Lj est la coordonnée de la couleur Cj selon l'axe L. Lj est comprise entre 0 et 100. $cm = \sum_{j=1}^{J} cj * qc_j$ où cj est la coordonnée de la couleur Cj selon l'axe C. cj est comprise entre 0 et 181. $Hpm = \sum_{j=1}^{J} PHpj * qc_j$ où PHpj est le poids d'indice p de la couleur Cj dans l'espace H et p = 1 à v. PHpj est compris entre 0 et 360.

Dans l'espace H des teintes (« Hue » en terminologie anglo-saxonne) comprises entre 0 et 360 ° on note :

$$H_1 = 0 \ ou \ 360 \ (coordonnées \ angulaires), \quad H_2 = {360}/{v}, H_3 = {2 * 360}/{v},$$

$$H_v = {(v - 1) * 360}/{v}$$

Pour une couleur donnée, il existe toujours une valeur x telle qu'une teinte Hx vérifie :
$x \in \{1,2, ... v\}$tel que $H_x \leq H < H_{x+1}$. Il faut noter que $(H_{v+1} = H_1)$ Et il existe toujours une valeur a comprise entre 0 et 1 telle que :

$$H_x * a + H_{x+1} * (1 - a) = H$$

Pour chaque couleur d'indice j, on a alors
$PH_{xj}$ = a et $PH_{(x+1)j}$ = 1 - a, et toutes les autres coordonnées $PH_{pj}$ d'indice p différent de x et x+1 sont nulles.

Les PH sont une expression de H qui est continue.

Les coordonnées moyennes Lm, cm et Hpm associées à une carte chromatiques sont influencées par les valeurs des descripteurs sensoriels de l'odeur correspondante.

Dans une série couleur Sb, l'impact des valeurs des descripteurs sensoriels sur les coordonnées moyennes suit une règle locale que l'on peut déterminer à l'aide d'au moins un deuxième réseau de neurones où les entrées sont des valeurs d'un ensemble de descripteurs sensoriels associées à une odeur ou un arôme et les sorties sont les coordonnées moyennes associées à cet arôme ou à cette odeur dans le repère L, H, C.

Nous ne faisons pas ici la description détaillée du deuxième réseau de neurones qui présente une architecture similaire à celle du premier réseau de neurones. La fonction d'activation de ces réseaux de neurones est la même que celle du premier réseau de neurones.

L'étape d'apprentissage 81 d'un deuxième réseau de neurones est réalisée de manière supervisée en fournissant, en entrée dudit deuxième réseau de neurones, les valeurs des ensembles de descripteurs sensoriels associées à des odeurs et/ou arômes test respectifs d'une série couleur considérée Sb et en tenant compte des descriptions colorimétriques associées aux odeurs et/ou arômes test respectifs de ces odeurs et /ou arômes test.

Le procédé comprend en outre une deuxième étape 82 d'analyse des valeurs de l'ensemble de descripteurs sensoriels associées à l'odeur ou l'arôme auquel on souhaite attribuer une description colorimétrique. Lors de cette étape 82, les valeurs de l'ensemble de descripteurs associées à l'odeur ou à l'arôme sont fournies en entrée dudit deuxième réseau de neurones, chaque deuxième réseau de neurones fournissant des coordonnées moyennes cibles, dans le repère L, C, H, d'une description colorimétrique cible associée à l'odeur ou à l'arôme considéré.

Un deuxième réseau de neurones d'indice b fournit les coordonnées moyennes cibles suivantes Lmbutb, cmbutb, Hpmbutb (p= 1 à v).

La description colorimétrique est générée à partir de la description théorique et à partir des coordonnées moyennes cibles obtenues.

On l'obtient de la manière suivante.

A partir des coordonnées moyennes cibles d'indice b : Lmbutb, cmbutb, Hpmbutb, obtenues à partir d'un deuxième réseau de neurones d'indice b, on calcule dans une étape 83, une nouvelle description colorimétrique DCN comprenant de nouvelles proportions colorimétriques qcnbj qui respectent les équations vérifiées par Lmbutb, cmbutb, Hpmbutb, à savoir :

$$Lmbutb = \sum_{j=1}^{J} Lj * qcnb_j$$

$$cmbutb = \sum_{j=1}^{J} cj * qcnb_j \, ,$$

$$Hpmbutb = \sum_{j=1}^{J} PHpj * qcnb_j$$

avec p = 1 à v,
et qui minimisent, pour au moins une couleur d'indice j, la valeur absolue $|qcnb_j - qcTb_j|$ de la différence entre la nouvelle proportion d'indice b et la proportion théorique d'indice b.

Avantageusement, le procédé comprend, préalablement à l'étape d'amélioration 80, une étape 73 de calcul de coefficients de similarité entre l'odeur ou l'arôme et des séries couleurs Sb respectives, à partir des valeurs de l'ensemble de descripteurs sensoriels attribuées à ladite odeur ou à l'arôme et à partir des valeurs de l'ensemble de descripteurs sensoriels attribuées aux odeurs et/ou arômes test regroupés dans les séries d'odeurs respectives et/ou à partir de la description colorimétrique théorique et à partir des description colorimétriques test attribués aux odeurs test des séries d'odeurs respectives.

Le procédé comprend ensuite une étape de vérification 74 dans laquelle on identifie, parmi les séries de couleurs, des séries couleurs dites séries couleurs similaires, avec lesquelles l'odeur ou l'arôme considéré vérifie un premier critère de similarité C1 prédéterminé consistant par exemple à identifier les séries couleurs avec lesquels l'odeur ou l'arôme présente un coefficient de similarité supérieur à un deuxième seuil S2 prédéterminé.

Cette étape 74 comprend également une étape consistant à vérifier si l'odeur ou l'arôme vérifie un deuxième critère de similarité C2 avec une série couleur particulière prise parmi les séries couleurs similaires, en vérifiant par exemple, si la différence entre le coefficient de similarité le plus élevé pris parmi les coefficients de similarité calculés avec les séries identifiées à l'étape précédente et le coefficient de similarité qui est juste inférieur à ce dernier est supérieure à un troisième seuil S3 prédéterminé.

Si oui, on soumet dans l'étape 82 les valeurs de l'ensemble de descripteurs sensoriels attribuées à l'odeur ou à l'arôme à un unique deuxième réseau de neurones préalablement soumis à une étape d'apprentissage 81 lors de laquelle on lui a fourni en entrée les valeurs de l'ensemble des descripteurs sensoriels associées aux odeurs et/ou arômes test regroupés dans la série couleur d'indice b qui a généré, avec l'odeur ou l'arôme, le coefficient de similarité le plus élevé (c'est-à-dire avec lequel l'odeur ou l'arôme vérifie le deuxième coefficient de similarité). En sortie du deuxième réseau de neurones, on obtient des coordonnées moyennes cibles d'ordre b dans le repère LCH : *Lmbutb, cmbutb, Hpmbutb.* On calcule ensuite dans une étape 84 la description colorimétrique à partir de la nouvelle description colorimétrique. La description colorimétrique est la nouvelle description colorimétrique qui comprend des nouvelles proportions qcn$_j$ associées à des couleurs C$_j$ qui vérifient les équations suivantes :

$$Lmbutb = \sum_{j=1}^{J} Lj * qcn_j$$

$$cmbutb = \frac{\sum_{j=1}^{J} cj * qcn_j}{J} ,$$

$$Hpmbutb = \sum_{j=1} PHpj * qcn_j$$

et minimisent, pour au moins une couleur Cj la valeur absolue $|qcn_j - qct_j|$ de la différence entre la nouvelle proportion d'ordre b et la proportion théorique.

Lorsque l'odeur ou l'arôme considéré ne présente pas de compatibilité privilégiée, cela signifie que l'odeur ou l'arôme considéré peut être compatible avec une pluralité de séries couleurs. L'odeur ou l'arôme vérifie le premier critère de similarité avec une pluralité de séries couleurs mais ne vérifie le deuxième critère de similarité avec aucune série couleur.

Dans ce cas, on soumet dans l'étape 82 les valeurs de l'ensemble de descripteurs sensoriels attribuées à l'odeur ou à l'arôme à une pluralité de deuxièmes réseau de neurones d'ordre b' (avec b'= 1 à B', où B' est égal au nombre de séries couleur identifiées lors de l'étape 73b).

Chaque réseau de neurones d'ordre b', avec b' = 1 à B' est préalablement soumit à une étape d'apprentissage 81 lors de laquelle on lui fournit en entrée les valeurs de l'ensemble des descripteurs sensoriels associées aux séries avec lesquelles l'odeur ou l'arôme considéré vérifie le premier critère de similarité.

Dans l'étape d'analyse 82, chaque réseau de neurones d'indice b', avec b' = 1 à B' reçoit en entrée les valeurs de l'ensemble des descripteurs sensoriels associés à l'odeur ou à l'arôme considéré de sorte à obtenir des coordonnées moyennes cibles d'ordre b' dans le repère LCH $Lmbutb'$, $cmbutb'$, $Hpmbutb'$, et à obtenir une nouvelle description colorimétrique d'ordre b' comprenant des nouvelles proportions colorimétriques d'ordre b' $qcnb'_j$ pour les couleurs d'indice j qui vérifient les équations suivantes :

$$Lmbutb' = \sum_{j=1}^{J} Lj * qcnb'_j$$

$$cmbutb' = \frac{\sum_{j=1}^{J} cj * qcnb'_j}{J} ,$$

$$Hpmbutb' = \sum_{j=1} PHpj * qcnb'_j$$

et qui minimisent pour au moins une couleur Cj la valeur absolue j $|qcnb'_j - qct_j|$ de la différence entre la nouvelle proportion d'ordre b' et la proportion théorique calculée pour la couleur d'indice.

On considère que l'odeur ou l'arôme considéré est une combinaison linéaire d'odeurs de séries différentes.

La description colorimétrique associée à l'odeur ou à l'arôme considéré comprend des proportions $qc_j$ associées à des couleurs $C_j$ respectives, les proportions colorimétriques associées aux couleurs étant des combinaisons linéaires des nouvelles proportions colorimétriques associées aux couleurs respectives. La proportion colorimétriques associée à une couleur d'indice j est égale à une combinaison linéaire des nouvelles proportions d'ordre b' $qcnb'_j$ et d'indice j c'est-à-dire :

$$qc_j = \sum_{b'=1}^{B'} qcnb'_j * kb' \text{ où } \sum_{b'=1}^{B'} kb' = 1$$

Autrement dit, la description colorimétrique obtenue est une combinaison linéaire des nouvelles descriptions colorimétriques obtenues pour les indices b' pour b' = 1 à B'.

Lorsque l'odeur ou l'arôme considéré n'est compatible avec aucune série couleur, c'est-à-dire lorsque le premier critère de similarité C1 n'est pas vérifié, soit l'odeur n'est pas analysable au moyen des bases de données actuelles (la seule solution est d'enrichir la base de données par de nouveaux odeurs et/ou arômes test), soit la description colorimétrique théorique est entachée d'erreur.

Dans ce dernier cas, le procédé comprend, préalablement à l'étape d'amélioration, une étape, de correction 75 de la description colorimétrique théorique DCT à partir d'une règle globale et à partir des valeurs de l'ensemble de descripteurs sensoriels attribuées l'odeur ou de l'arôme considéré.

Connaissant les descripteurs sensoriels de l'odeur, on peut obtenir les probabilités d'occurrence de chacune des couleurs Cj dans les descriptions colorimétriques des odeurs test avec chacun de ces descripteurs.

Il est possible de construire, à partir des valeurs de l'ensemble de descripteurs sensoriels et des descriptions colorimétriques test associées aux odeurs et/ou arômes test respectifs, une règle globale permettant de calculer pour au moins une couleur, à partir de valeurs d'un ensemble de descripteurs sensoriels comprenant au moins un descripteur sensoriel une probabilité de présence, dans une description colorimétrique associée à ces valeurs, d'une proportion colorimétrique non nulle associée à ladite couleur..

L'étape de correction comprend donc :

- une étape de calcul, pour chaque couleur de l'ensemble de couleurs, à partir des valeurs de l'ensemble de descripteurs sensoriels associées à l'odeur ou à l'arôme considéré, et à partir de la règle globale, de la probabilité que la proportion colorimétrique associée à ladite couleur soit non nulle,
- une étape de mise à jour de la description colorimétrique théorique, en annulant les proportions associés aux couleurs pour lesquelles la probabilité que la proportion associée à cette couleur soit non nulle est inférieure à un quatrième seuil prédéterminé, et de réajustement des autres proportions en conservant le même rapport entre ces autres proportions et de façon que la somme de ces autres proportions réajustées soit égale à 1.

A l'issu de cette étape de correction, on retourne à l'étape 73 de calcul de coefficients de similarité entre l'odeur et des séries couleurs.

Le procédé selon l'invention permet de transcrire de manière automatique, objective et reproductible une odeur ou un arôme en une description colorimétrique à partir d'associations entre des couleurs et des odeurs qui sont réalisées préalablement. Le fait d'utiliser des coefficients de corrélation entre des couleurs ou combinaisons de couleur et des molécules et/ou des combinaisons de molécules pour réaliser cette transcription permet d'obtenir une bonne fiabilité des résultats. Cela permet également de pouvoir transcrire une odeur ou un arôme qui ne fait pas partie des échantillons d'odeur et/ou d'arôme qui ont été préalablement testés par des juges. Par ailleurs, il permet d'attribuer des descriptions colorimétriques à des odeurs ou arômes complexes issues d'un nombre important de molécules volatiles en ayant effectué des tests préalables auprès des juges sur des odeurs et/ou arômes simples issus d'un faible nombre de molécules volatiles. Les réseaux de neurones de par leurs capacités d'apprentissage et de classification sont particulièrement adaptés aux problèmes de nature statistique comme celui de l'invention.

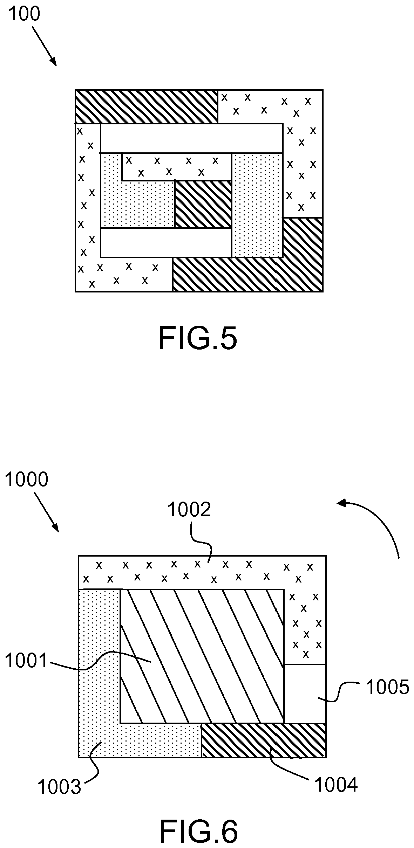

Le procédé selon l'invention comprend en outre avantageusement une étape de représentation 90 de l'odeur ou de l'arôme O au moyen d'une carte ou profil chromatique dans laquelle le pourcentage de la surface de la carte chromatique occupé par une couleur Cj de l'ensemble de couleurs correspond au rapport entre la proportion associée à ladite couleur $qc_j$ dans la description colorimétrique et la somme des proportions associées à un sous-ensemble de l'ensemble de couleurs dans la description colorimétrique, le sous-ensemble étant choisi de manière à ce que la somme des proportions associées aux couleurs qu'il contient, dans la description colorimétrique, est au moins égale à un seuil prédéterminé qui est, par exemple, égal à 95%. Si le seuil est de 100% le sous-ensemble est l'ensemble de couleurs. Sur la figure 5, on a représenté un exemple de carte chromatique 100 comprenant une pluralité de zones de différentes couleurs. Les zones présentant des couleurs différentes sont représentées par des motifs différents. La proportion de la surface de la carte chromatique présentant une couleur déterminée, c'est-à-dire associée à un motif, est égale à la proportion associée à cette couleur dans la description colorimétrique. Sur la figure 5, la carte chromatique présente des zones associées à 4 couleurs (ou motifs) différentes. La description colorimétrique comprend donc des proportions non nulles pour uniquement 4 couleurs.

Sur cet exemple, la carte chromatique comprend une pluralité de zones associée à chaque couleur mais on pourrait prévoir une unique zone, sur la carte chromatique présentant une couleur prise parmi les couleurs présentant des proportions non nulle dans la description colorimétrique.

On pourrait aussi prévoir une carte chromatique sous forme d'un diagramme circulaire ou tout autre forme du moment qu'elle présente au moins une zone uniforme présentant une couleur déterminée. La proportion de la surface de la carte chromatique occupée par la ou les zones présentant une couleur déterminée est égale à la proportion associée à cette couleur dans la description colorimétrique.

Un mode de construction d'une carte chromatique comprenant une zone centrale et une zone périphérique entourant la zone centrale peut consister à faire apparaitre au centre de la carte la couleur présentant la plus grande proportion au centre de la carte et à faire apparaitre les autres couleurs autour de la première en tournant autour de la zone centrale dans un sens (horaire ou anti-horaire) selon un ordre correspondant à l'ordre décroissant des proportions associées auxdites couleurs. Les proportions respectives de la surface de la carte occupées par les couleurs respectives correspondent aux proportions respectives desdites couleurs dans la description colorimétrique. La figure 6 représente un exemple de représentation 1000 de ce type, réalisée en tournant dans le sens antihoraire, représenté par la flèche, dans lequel la couleur 1001 présente la plus grande proportion dans la description colorimétrique et occupe la plus grande proportion de la surface de carte 1000. Les couleurs 1002, 1003, 1004 et 1005 occupent des proportions respectives de la surface de la carte 1000 de plus en plus faibles.

Avantageusement, la première étape d'analyse physico-chimique comprend une étape d'attribution d'un premier vecteur physico-chimique comprenant des proportions associées à un premier ensemble de molécules respectives, comprenant les molécules de l'ensemble de molécules ainsi que des molécules supplémentaires. Cette première étape comprend en outre une étape de filtrage consistant à construire le vecteur physico-chimique à partir du premier vecteur physico-chimique.

Il peut en être de même pour la deuxième étape d'analyse. Autrement dit, cette deuxième étape peut comprendre une étape d'attribution d'un premier vecteur physico-chimique test à chaque odeur ou arôme test comprenant des proportions test associées à un premier ensemble de molécules respectives, comprenant les molécules de l'ensemble de molécules ainsi que des molécules supplémentaires, ainsi qu'une étape de filtrage consistant à construire chaque vecteur physico-chimique test associé à une odeur ou à un arôme à partir du premier vecteur physico-chimique test associé à l'odeur ou à l'arôme.

L'invention a également pour objet un procédé de transcription, en une description physico-chimique de résultat comprenant une liste de molécules, d'une description initiale comprenant une description colorimétrique initiale DCI comprenant des proportions initiales $qcI_j$ associées à un ensemble de couleurs respectives Cj, et éventuellement de valeurs, dite initiales, d'un ensemble de descripteurs sensoriels (tel que décrit précédemment). Ces valeurs initiales correspondent à des notes sensorielles relatives à l'odeur ou à l'arôme que l'on recherche.

Ce procédé comprend une étape d'analyse physico-chimique d'un ensemble d'odeurs et/ou d'arômes Ot dits de test afin d'attribuer à chaque odeur ou arôme test Ot dudit ensemble, une description physico-chimique dite de test $PC_t$ comprenant un vecteur physico-chimique dit de test $PQ_t$ comprenant des proportions test $qmt_i$ associées audit ensemble prédéterminé de molécules volatiles respectives M correspondant à l'étape 20 précédemment décrite.

Ce procédé comprend également une première étape d'attribution, aux odeurs et/ou arômes test Ot de descriptions colorimétriques test DCt respectives comprenant des proportions dites test $qct_j$ associées à un ensemble de couleurs respectives Cj. Cette étape correspond à l'étape 30 précédemment décrite et peut être réalisée conformément aux étapes 30a et 30b.

Ce procédé comprend également une deuxième étape d'attribution, d'une description colorimétrique hypothétique comprenant des proportions colorimétriques hypothétiques qcHj associées à l'ensemble de couleurs respectives Cj, à un vecteur physico-chimique hypothétique PCH comprenant des proportions hypothétiques $qmH_j$ associées audit ensemble de molécules volatiles respectives Mi, cette étape d'attribution étant réalisée au moyen du premier réseau de neurones 1 précédemment décrit. Ce réseau de neurones a préalablement subi une première phase d'apprentissage identique à l'étape 60 précédemment décrite, réalisée de manière à fixer les poids du réseau de neurones.

La deuxième étape d'attribution est réalisée en présentant, en entrée du premier réseau de neurones, un vecteur physico-chimique hypothétique PCH associées audit ensemble de molécules volatiles respectives Mi, ledit premier réseau de neurones 1 générant en sortie, ladite description colorimétrique hypothétique DCH.

Le procédé comprend également une étape de calcul d'erreur dans laquelle on calcule une erreur représentative d'un écart entre la description colorimétrique hypothétique et la description colorimétrique initiale, ladite étape de calcul d'erreur étant suivie d'une étape de mise à jour du vecteur physico-chimique hypothétique PCH, à partir de l'erreur et du vecteur physico-chimique hypothétique (de façon à engendrer une diminution de cette erreur lors de la présentation du vecteur physico-chimique hypothétique mise à jour en entrée du premier réseau de neurones) et de retour à l'étape d'attribution tant que l'erreur est supérieure à un seuil d'erreur prédéterminé, la liste des molécules étant déterminée à partir du vecteur physico-chimique hypothétique qui engendre une erreur de calcul inférieure ou égale à l'erreur.

Il y a plusieurs manières de déterminer un vecteur physico-chimique hypothétique.

Une première manière peut consister à utiliser des valeurs dites initiales d'un ensemble de descripteurs sensoriels.

L'ensemble de descripteurs sensoriels est un ensemble tel que décrit précédemment.

Le procédé comprend avantageusement, préalablement à l'étape de détermination du vecteur physico-chimique hypothétique :

- une étape de construction d'une autre loi globale permettant de déterminer, à partir des valeurs d'un ensemble de descripteurs sensoriels associées à une odeur ou un arôme O, les probabilités de présence c'est-à-dire les probabilités que les proportions respectives associées à un ensemble de molécules respectives dans le mélange de molécules volatiles à l'origine de l'odeur ou de l'arôme O soient non nulles, cette étape étant réalisée à partir des vecteurs physico-chimiques test et des valeurs de l'ensemble de descripteurs sensoriels associés aux odeurs et/ou arômes test,

- une étape de regroupement des odeurs et/ou arômes test dans un ensemble de séries couleurs Sb d'ordre b par similarité des leurs descriptions colorimétriques test respectives correspondant à l'étape 72 respectives.

L'étape de détermination du vecteur physico-chimique hypothétique est réalisée notamment à partir des valeurs dites initiales d'un ensemble de descripteurs sensoriels, de la loi globale, des valeurs dudit ensemble de descripteurs sensoriels associées aux odeurs et/ou arômes test, des descriptions colorimétriques associées aux odeurs et/ou arômes test et des vecteurs physico-chimiques test.

Cette étape comprend :

- une étape de détermination de probabilités de présence, dans l'odeur ou l'arôme résultat, de l'ensemble des molécules respectives, cette étape étant réalisée à partir des valeurs initiales de l'ensemble de descripteurs sensoriels et à partir de l'autre loi globale,
- une étape d'établissement d'une liste de molécules possibles prises dans l'ensemble de molécules, cette liste de molécules possibles correspondant aux molécules qui, parmi les molécules de l'ensemble de molécules, présentent une probabilité de présence non nulle,
- une étape de calcul de coefficients de corrélation entre la description initiale DI et les séries couleurs respectives à partir des valeurs initiales de l'ensemble de descripteurs sensoriels et des valeurs de l'ensemble de descripteurs sensoriels associées aux odeurs et/ou arômes test respectifs ainsi qu'à partir des descriptions colorimétriques test et initiales),
- une étape de vérification lors de laquelle on identifie, parmi les séries couleurs, les séries couleurs avec lesquelles la description initiale vérifie un critère de corrélation prédéterminé, cette étape est réalisée à partir des coefficients de similarité cités au paragraphe précédent,
- une étape d'élaboration du vecteur physico-chimique hypothétique à partir de la liste de molécules possibles et à partir des vecteurs physico-chimiques test associés aux odeurs et/ou arômes test compris dans les séries couleurs avec lesquelles la description initiale vérifie le critère de corrélation. Le vecteur physico-chimique hypothétique comprend des proportions hypothétiques $qmH_i$ associées aux molécules Mi possibles respectives.

Le premier vecteur physico-chimique hypothétique que l'on présente au premier réseau de neurones est critique pour que le système propose au final un vecteur physico-chimique hypothétique acceptable.

Il peut être nécessaire de présenter plusieurs vecteurs physico-chimiques hypothétiques avant d'obtenir une solution acceptable. A cet effet, l'étape de calcul d'erreur est suivie, par exemple, lorsque l'erreur est supérieure à un seuil prédéterminé, d'une étape de retour à l'étape d'établissement d'un nouveau vecteur physico-chimique hypothétique puis à l'étape de présentation de celui-ci au premier réseau de neurones.

Dans une condition extrême, où on ne trouve aucune série couleur corrélée à la description initiale, on peut établir un troisième réseau de neurones du type perceptron multicouches comprenant une couche d'entrée apte à recevoir des coordonnées de couleur dans un repère LCH et des descripteurs sensoriels, et à fournir en sortie un vecteur physico-chimique.

La sortie fournie par ce réseau peut être utilisée en tant que vecteur physico-chimique hypothétique. Cette méthode présente un risque d'erreur accru du fait de la différence de dimension des deux types de données physico-chimiques et sensorielles.

Ce deuxième procédé permet de proposer une liste de molécules olfactives correspondant à des notes sensorielles prédéterminées. Ces molécules pourront ainsi être arrangées par un aromaticien ou un parfumeur afin de délivrer un parfum en adéquation avec une charte colorée, par exemple.

Avantageusement, au moins une partie des étapes 40, 50, 60, 70 et, de préférence toutes ces étapes, sont réalisées au moyen d'un calculateur, c'est-à-dire par ordinateur. Avantageusement, l'étape 90 est commandée par un calculateur. Avantageusement, dans le deuxième procédé, au moins une des étapes suivantes et de préférences toutes les étapes suivantes sont mises en oeuvre par un calculateur : la deuxième étape d'attribution de description colorimétrique, la première étape d'apprentissage, l'étape de calcul d'erreur, l'étape de mise à jour du vecteur physico-chimique. Avantageusement, l'étape préalable de détermination du vecteur physico-chimique hypothétique est mise en oeuvre par un calculateur. Avantageusement, l'étape de construction d'une autre loi globale et l'étape de regroupement des odeurs et/ou arômes test sont mises en oeuvre par un calculateur.

**Revendications**

1. Procédé de transcription d'une odeur ou d'un arôme (O) en une description colorimétrique (DC) comprenant les étapes suivantes :

   - une première étape (10) d'analyse physico-chimique de ladite odeur ou dudit arôme (O) afin de lui associer une description physico-chimique (PCN) comprenant un vecteur physico-chimique (PQ), comprenant des proportions ($qm_i$) associées à un ensemble prédéterminé de molécules volatiles respectives (Mi),
   - une deuxième étape (20) d'analyse physico-chimique d'un ensemble d'odeurs et/ou d'arômes (Ot) dits de test afin d'attribuer à chaque odeur ou arôme test (Ot) dudit ensemble, une description physico-chimique dite de test ($PC_t$) comprenant un vecteur physico-chimique dit de test ($PQ_t$) comprenant des proportions test ($qmt_i$) associées audit ensemble prédéterminé de molécules volatiles respectives (Mi),
   - une étape (30) d'attribution, aux odeurs et/ou arômes test (Ot) de descriptions colorimétriques test (DCt) respectives comprenant des proportions dites test ($qct_j$) associées à un ensemble de couleurs respectives (Cj),
   - une étape d'attribution (70), à l'odeur ou à l'arôme (O), d'une description colorimétrique comprenant des proportions ($qc_j$) associées auxdites couleurs (Cj) respectives, ladite étape d'attribution (70) comprenant une première étape (71) d'attribution, à l'odeur ou à l'arôme, d'une description colorimétrique théorique (DCT) comprenant des proportions colorimétriques théoriques ($qcT_j$) associées auxdites couleurs (Cj) respectives, cette première étape d'attribution (71) étant réalisée au moyen d'un premier réseau de neurones artificiels (1) présentant une architecture en couches, en lui présentant, en entrée le vecteur physico-chimique (PQ), ledit premier réseau de neurones (1) générant en sortie, ladite description colorimétrique théorique (DCT), ledit premier réseau de neurones (1) étant préalablement soumis à une première étape d'apprentissage (60) lors de laquelle on lui fournit, en entrée, des vecteurs physico-chimiques (PQt) associés à l'ensemble d'odeurs et/ou d'arômes test, ladite première étape d'apprentissage étant réalisée de manière supervisée en tenant compte des descriptions colorimétriques test associées à l'ensemble d'odeurs et/ou d'arômes test respectifs.

2. Procédé de transcription selon la revendication précédente, comprenant, préalablement à la première étape d'apprentissage (60) une étape de fixation (50) lors de laquelle on fixe des poids de premières liaisons synaptiques reliant deux à deux des neurones dudit réseau de neurones (1) de façon que les poids associées aux premières liaisons synaptiques ne soient pas modifiables lors de l'étape d'apprentissage.

3. Procédé de transcription selon la revendication précédente, dans lequel l'étape de fixation (50) est réalisée à partir de premiers coefficients de corrélation, chaque premier coefficient de corrélation étant calculé entre une molécule (Mi) de l'ensemble de molécules et une couleur de l'ensemble de couleurs (Cj), calculés dans une étape préalable (40) à partir de vecteurs physico-chimiques test et des descriptions colorimétriques (DCt) associées aux odeurs et/ou arômes test (Ot) auxquels lesdits vecteurs physico-chimiques sont attribués.

4. Procédé de transcription selon la revendication précédente, dans lequel l'étape préalable (40) comprend, pour au moins une couleur d'indice j (Cj) :

   - une étape de sélection (401), dans l'ensemble d'odeurs et/ou arômes test (Ot), d'un sous-ensemble (SEj) d'indice j d'odeurs et/ou d'arômes test qui sont associés à des descriptions colorimétriques test (DCt) présentant des proportions non nulles pour ladite couleur d'indice j,
   - une première étape de regroupement (402) des odeurs et/ou arômes test du sous ensemble (SEj) dans un ensemble de groupes d'odeurs et/ou d'arômes test, de façon que les vecteurs physico-chimiques (PQt) associés aux odeurs et/ou arômes test présents dans un groupe présentent entre eux des premiers coefficients de corrélation plus importants qu'avec les vecteurs physico-chimiques (PQt) associés aux odeurs et/ou arômes test présents dans les autres groupes dudit ensemble de groupes,
   - et, pour au moins un desdits groupes,

     - une étape (403) d'élaboration d'une liste de molécules communes correspondant aux molécules dont la proportion test est non nulle dans tous les vecteurs physico-chimiques test associés aux odeurs et/ou arômes test regroupés dans ledit groupe,
     - et, pour au moins une molécule prise dans la liste de molécules communes,

       - étape (404) lors de laquelle on établit une première suite (SU1) dans laquelle on ordonne les odeurs et/ou arômes test regroupés dans ledit groupe selon l'ordre croissant de la proportion de ladite molécule dans les vecteurs physico-chimiques test associés auxdites odeurs et/ou arômes test,

- une étape (405) lors de laquelle on établit une deuxième suite (SU2) dans laquelle on ordonne les odeurs et/ou arômes test regroupés dans ledit groupe selon l'ordre croissant de la proportion de ladite couleur dans les descriptions colorimétriques associées auxdites odeurs et/ou arômes test,
- une étape (406) de calcul d'un autre coefficient de corrélation entre la première suite (SU1) et la deuxième suite (SU2),

- une étape (407) de calcul d'au moins un premier coefficient de corrélation entre une molécule de l'ensemble de molécules et une couleur de l'ensemble de couleurs à partir des autres coefficients de corrélation calculés pour ladite molécule et ladite couleur.

5.  Procédé de transcription selon l'une quelconque des revendications précédentes, dans lequel la description colorimétrique (DC) est la description colorimétrique théorique (DCT).

6.  Procédé de transcription selon l'une quelconque des revendications 1 à 4, dans lequel l'étape (70) d'attribution de la description colorimétrique comprend une étape d'amélioration (80) de la description colorimétrique théorique à partir de valeurs d'un ensemble de descripteurs sensoriels attribuées à l'odeur ou à l'arôme et à des odeurs et/ou arômes test respectifs de façon à obtenir ladite description colorimétrique, l'ensemble de descripteurs sensoriels comprenant au moins un descripteur sensoriel pris parmi une source d'une odeur ou d'un arôme, un indice de comestibilité, un indice d'agréabilité, un indice de familiarité, une note olfactive, un indice d'intensité.

7.  Procédé de transcription selon la revendication précédente, dans lequel l'étape d'amélioration (80) comprend :

- une étape (81) de soumission d'au moins un deuxième réseau de neurones artificiels d'indice b à une deuxième étape d'apprentissage, un deuxième réseau de neurones présentant une architecture en couches comprenant une couche d'entrée apte à recevoir des valeurs associées à l'ensemble de descripteurs sensoriels et fournissant en sortie des première Lm, deuxième cm et troisièmes Hpm coordonnées moyennes, où p = est un entier allant de 1 à v entier, dans un repère colorimétrique dit L, C, H, ledit deuxième réseau de neurones recevant, lors de la deuxième étape d'apprentissage, des valeurs de l'ensemble de descripteurs sensoriels associées aux odeurs et/ou arômes test présents dans une série couleur constituée d'au moins une partie des odeurs et/ou arômes test de l'ensemble d'odeurs et/ou arômes test,
- une deuxième étape d'analyse (82) de l'odeur ou de l'arôme en fournissant, les valeurs de l'ensemble de descripteurs sensoriels en entrée dudit deuxième réseau de neurone d'indice b qui fournit en sortie, des première Lmbutb, deuxième cmbutb et troisièmes Hpmbutb coordonnées moyennes cibles d'indice b dans le repère L, H, C, d'une description colorimétrique cible associée à l'odeur ou à l'arôme considéré,
- une étape de calcul (83), d'une nouvelle description colorimétrique comprenant de nouvelles proportions $qcnb_j$ qui respectent, quelque soit j compris entre 1 et J, les équations suivantes :

$$Lmbutb = \sum_{j=1}^{J} Lj * qcnb_j$$

$$cmbutb = \sum_{j=1}^{J} cj * qcnb_j \, ,$$

$$Hpmbutb = \sum_{j=1}^{J} PHpj * qcnb_j$$

avec p entier allant de 1 à v et v entier, où Lj, cj et PHpj sont les coordonnées respectives de la couleur Cj d'indice j dans le repère L, H, C.
et qui minimisent, pour au moins une couleur Cj, la valeur absolue $|qcnb_j - qcTb_j|$ de la différence entre la nouvelle proportion d'ordre b et la proportion théorique d'ordre b.

8.  Procédé de transcription selon l'une quelconque des revendications 6 à 7, comprenant, préalablement à l'étape d'amélioration (80), une deuxième étape de regroupement (72) d'odeurs et/ou d'arômes test appartenant à l'ensemble des odeurs et/ou arômes test (Ot) dans un ensemble de séries dites séries couleurs, par similarité des descriptions colorimétriques test (DCt) qui leurs sont attribuées.

**9.** Procédé de transcription selon la revendication précédente, comprenant :

- une étape de calcul de coefficients de similarité (73) entre l'odeur et les séries couleurs respectives, à partir des valeurs de l'ensemble de descripteurs sensoriels attribuées à l'odeur ou à l'arôme et aux odeurs et/ou arômes test regroupés dans des séries couleurs respectives et/ou à partir de la description colorimétrique théorique et à partir des descriptions colorimétriques test attribués aux odeurs test regroupées dans les séries d'odeurs respectives,
- une étape de vérification (74) lors de laquelle on identifie, parmi les séries couleurs, des séries couleurs similaires avec lesquelles l'odeur ou l'arôme vérifie un premier critère de similarité prédéterminé, et lors de laquelle on vérifie si l'odeur ou l'arôme vérifie un deuxième critère de similarité avec une série couleur prise parmi les séries couleurs similaires,
- et, lorsque l'odeur ou l'arôme vérifie le premier et le deuxième critère de similarité, on soumet, lors de la deuxième étape d'analyse (82) les valeurs de l'ensemble de descripteurs sensoriels attribués à l'odeur ou à l'arôme à un unique deuxième réseau de neurones préalablement soumis à l'étape d'apprentissage (81) lors de laquelle on lui fournit en entrée les valeurs de l'ensemble de descripteurs sensoriels attribuées aux odeurs et/ou arômes test regroupés dans ladite série couleur avec laquelle l'odeur ou l'arôme vérifie le deuxième critère de similarité,
- et, lorsque l'odeur ou l'arôme vérifie le premier critère de similarité mais pas le deuxième critère de similarité, on soumet, lors de la deuxième étape d'analyse (82), les valeurs de l'ensemble de descripteurs sensoriels attribués à l'odeur ou à l'arome à une pluralité de deuxième réseau de neurones préalablement soumis à l'étape d'apprentissage (81) lors de laquelle on lui fournit en entrée les valeurs de l'ensemble de descripteurs sensoriels attribuées aux odeurs et/ou arômes test regroupés dans les séries couleur avec laquelle l'odeur ou l'arôme vérifie le premier critère de similarité, les proportions ($qc_j$) colorimétriques associées auxdites couleurs respectives étant des combinaisons linéaires des nouvelles proportions colorimétriques associées aux couleurs respectives.

**10.** Procédé de transcription selon l'une quelconque des revendications 6 à 9, comprenant, préalablement à l'étape d'amélioration (80), une étape de correction (75) consistant à mettre à jour la description colorimétrique théorique (DCT) à partir des valeurs de l'ensemble de descripteurs associés à l'odeur ou à l'arôme et à partir d'une règle globale permettant de calculer, pour au moins une couleur, et à partir de valeurs de l'ensemble de descripteurs sensoriels attribués à l'arôme ou à l'odeur, une probabilité de présence, dans une description colorimétrique associée à ces valeurs, d'une proportion colorimétrique associée à ladite couleur, ladite étape de correction (75) étant suivie d'un retour à l'étape de calcul de coefficients de similarité (73).

**11.** Procédé de transcription selon l'une quelconque des revendications précédentes, dans lequel l'étape (30) d'attribution, aux odeurs et/ou arômes test, d'une description colorimétrique comprenant des proportions test ($qct_j$) associées à un ensemble de couleurs respectives (Cj), comprend :

- une étape d'attribution (30a) de couleurs dites couleurs jugées à des odeurs et/ou arômes de test (Ot) par un ensemble de juges humains,
- une étape d'élaboration (30b) d'une description colorimétrique dite de test de chaque odeur ou arôme test (Ot) comprenant des proportions test ($qct_j$) associées à un ensemble de couleurs respectives (Cj) à partir de la proportion de juges qui, parmi les juges appartenant à un autre ensemble de juges comprenant tout ou partie dudit ensemble de juges humains, ont attribué chacune des couleurs jugées à ladite odeur ou audit arôme de test (Ot).

**12.** Procédé de transcription selon la revendication précédente, dans lequel les proportions test ($qct_j$) relatives aux différentes couleurs ($C_j$) sont déterminées à partir de la proportion de juges qui, parmi les juges appartenant à l'autre ensemble de juges, ont attribué chacune des couleurs jugées audit arôme ou à ladite odeur et à partir de coefficients de proximité calculés entre la couleur jugée et les couleurs de l'ensemble de couleurs.

**13.** Procédé de transcription selon l'une quelconque des revendications précédentes, comprenant une étape de représentation (90) de la nouvelle odeur ou du nouvel arôme (O) au moyen d'une carte chromatique dans laquelle le pourcentage de la surface de la carte chromatique occupé par une couleur (Cj) de l'ensemble de couleurs correspond au rapport entre proportion associée à ladite couleur ($qc_j$) dans la description colorimétrique et la somme des proportions associées à un sous-ensemble de l'ensemble de couleurs dans la description colorimétrique, le sous-ensemble étant choisi de manière à ce que la somme des proportions associées aux couleurs qu'il contient, dans la description colorimétrique, est au moins égale à un seuil prédéterminé.

14. Procédé de transcription d'une description colorimétrique initiale comprenant des proportions colorimétriques initiales relatives à un ensemble de couleurs respectives en une description physico-chimique de résultat comprenant un ensemble prédéterminé de molécules volatiles comprenant les étapes suivantes :

- une étape d'analyse physico-chimique d'un ensemble d'odeurs et/ou d'arômes dits de test afin d'attribuer à chaque odeur ou arôme test dudit ensemble, une description physico-chimique dite de test comprenant un vecteur physico-chimique dit de test comprenant des proportions test associées audit ensemble prédéterminé de molécules volatiles respectives,
- une première étape d'attribution, aux odeurs et/ou arômes test de descriptions colorimétriques test respectives comprenant des proportions dites test associées à un ensemble de couleurs respectives,
- une deuxième étape d'attribution, d'une description colorimétrique hypothétique comprenant des proportions colorimétriques hypothétiques à l'ensemble de couleurs respectives, à un vecteur physico-chimique hypothétique comprenant des proportions hypothétiques associées audit ensemble de molécules volatiles respectives, cette deuxième étape d'attribution étant réalisée au moyen d'un premier réseau de neurones artificiels présentant une architecture en couches, en lui présentant, en entrée un vecteur physico-chimique hypothétique comprenant des proportions hypothétiques associées audit ensemble de molécules volatiles respectives, ledit premier réseau de neurones générant en sortie, ladite description colorimétrique hypothétique, ledit premier réseau de neurones étant préalablement soumis à une première étape d'apprentissage lors de laquelle on lui fournit, en entrée, des vecteurs physico-chimiques associés à l'ensemble d'odeurs et/ou d'arômes test, ladite première étape d'apprentissage étant réalisée de manière supervisée en tenant compte des descriptions colorimétriques test associées à l'ensemble d'odeurs et/ou d'arômes test respectifs,
- une étape de calcul d'erreur dans laquelle on calcule une erreur représentative d'un écart entre la description colorimétrique hypothétique et la description colorimétrique initiale, ladite étape de calcul d'erreur étant suivie d'une étape de mise à jour du vecteur physico-chimique hypothétique, à partir de l'erreur et du vecteur physico-chimique hypothétique, et de retour à l'étape d'attribution tant que l'erreur est supérieure à un seuil d'erreur prédéterminé, la description physico-chimique de résultat étant déterminée à partir du vecteur physico-chimique hypothétique qui engendre une erreur de calcul inférieure ou égale audit seuil d'erreur.

15. Procédé de transcription selon la revendication précédente, dans lequel la description initiale est associée à des valeurs dites initiales d'un ensemble de descripteurs sensoriels et les odeurs et/ou arômes test sont associés à des valeurs dudit ensemble de descripteurs sensoriels, ledit ensemble de descripteurs sensoriels comprend au moins un descripteur sensoriel pris parmi une source d'une odeur ou d'un arôme, un indice de comestibilité, un indice d'agréabilité, un indice de familiarité, une note olfactive, un indice d'intensité, et comprenant une étape préalable de détermination du vecteur physico-chimique hypothétique à partir des valeurs dites initiales de l'ensemble de descripteurs sensoriels et à partir des valeurs dudit ensemble de descripteurs sensoriels associés à l'odeur ou à l'arôme test.

16. Procédé de transcription selon la revendication précédente, comprenant, préalablement à l'étape de détermination du vecteur physico-chimique hypothétique :

- une étape de construction d'une autre loi globale permettant de déterminer, à partir des valeurs d'un ensemble de descripteurs sensoriels associées à une odeur ou un arôme , les probabilités de présence de l'ensemble de molécules respectives dans l'odeur ou de l'arôme O soient non nulles, cette étape étant réalisée à partir des vecteurs physico-chimiques test et des valeurs de l'ensemble de descripteurs sensoriels associés aux odeurs et/ou arômes test,
- une étape de regroupement des odeurs et/ou arômes test dans un ensemble de séries couleurs par similarité des leurs descriptions colorimétriques test respectives,

l'étape de détermination du vecteur physico-chimique hypothétique comprenant :

- une étape de détermination de probabilités de présence, dans l'odeur ou l'arôme résultat, de l'ensemble des molécules respectives, cette étape étant réalisée à partir des valeurs initiales de l'ensemble de descripteurs sensoriels et à partir de l'autre loi globale,
- une étape d'établissement d'une liste de molécules possibles prises dans l'ensemble de molécules, cette liste de molécules possibles correspondant aux molécules qui, parmi les molécules de l'ensemble de molécules, présentent une probabilité de présence non nulle,
- une étape de calcul de coefficients de corrélation entre la description initiale et les séries couleurs respectives à partir des valeurs initiales de l'ensemble de descripteurs sensoriels et des valeurs de l'ensemble de descripteurs sensoriels associées aux odeurs et/ou arômes test respectifs ainsi qu'à partir des descriptions colorimétriques test et initiales,

- une étape de vérification lors de laquelle on identifie, parmi les séries couleurs, les séries couleurs avec lesquelles la description initiale vérifie un critère de corrélation prédéterminé, cette étape est réalisée à partir des coefficients de similarité cités au paragraphe précédent,

- une étape d'élaboration du vecteur physico-chimique hypothétique à partir de la liste de molécules possibles et à partir des vecteurs physico-chimiques test associés aux odeurs et/ou arômes test compris dans les séries couleurs avec lesquelles la description initiale vérifie le critère de corrélation, le vecteur physico-chimique hypothétique comprenant des proportions hypothétiques associées aux molécules Mi possibles respectives.

17. Procédé selon l'une quelconque des revendications 14 à 16, dans lequel les étapes suivantes sont mises en oeuvre par un calculateur : la deuxième étape d'attribution d'une description colorimétrique, la première étape d'apprentissage, l'étape de calcul d'erreur, l'étape de mise à jour du vecteur physico-chimique.

## Patentansprüche

1. Verfahren zum Übertragen eines Geruches oder Aromas (O) in eine kolorimetrische Beschreibung (DC), umfassend die Schritte:

- einen ersten Schritt (10) des Durchführens einer physikalisch-chemischen Analyse des Geruches bzw. Aromas (O), um diesem eine physikalisch-chemische Beschreibung (PCN) zuzuordnen, die einen physikalisch-chemischen Vektor (PQ) umfasst, der Anteile ($qm_i$), die mit einer vorgegebenen Gruppe aus jeweiligen flüchtigen Molekülen (Mi) zusammenhängen, umfasst,

- einen zweiten Schritt (20) des Durchführens einer physikalisch-chemischen Analyse einer Gruppe von sogenannten Prüfgerüchen und/oder -Aromen (Ot) um jedem Prüfgeruch bzw. -aroma (Ot) der Gruppe eine sogenannte physikalisch-chemische Prüfbeschreibung ($PC_t$) zuzuordnen, die einen sog. physikalisch-chemischen Prüfvektor ($PQ_t$) umfasst, umfassend Prüfanteile ($qmt_i$), die mit der vorgegebenen Gruppe von jeweiligen flüchtigen Molekülen (Mi) zusammenhängen,

- einen Schritt (30) des Zuordnens jeweiliger kolorimetrischer Prüfbeschreibungen (DCt) zu den Prüfgerüchen und/oder -aromen (Ot), umfassend sog. Prüfanteile ($qct_j$), die mit einer Gruppe von jeweiligen Farben (Cj) zusammenhängen,

- einen Schritt (70) des Zuordnens einer die mit den jeweiligen Farben (Cj) zusammenhängenden Anteile ($qc_j$) umfassenden kolorimetrischen Beschreibung zum Geruch bzw. Aroma (O), wobei das Zuordnen (70) umfasst: in einem ersten Schritt: Zuordnen (71) einer die den jeweiligen Farben (Cj) zusammenhängende theoretische Anteile ($qcTj$) umfassenden theoretischen kolorimetrischen Beschreibung (DCT) zum Geruch bzw. Aroma, wobei dieser erste Zuordnungsschritt (71) mittels eines ersten künstlichen neuronalen Netzes (1) mit mehrschichtiger Architektur erfolgt, indem man ihm am Eingang den physikalisch-chemischen Vektor (PQ) präsentiert, wobei das erste neuronale Netz (1) am Ausgang die theoretische kolorimetrische Beschreibung (DCT) erzeugt, wobei das erste neuronale Netz (1) im Voraus einem ersten Lernschritt (60) unterzogen wird, bei dem man ihm am Eingang physikalisch-chemische Vektoren (PQt), die mit der Gruppe von Prüfgerüchen und/oder -aromen zusammenhängen, zuführt, wobei der erste Lernschritt unter Aufsicht erfolgt und dabei die mit der Gruppe der jeweiligen Prüfgerüche und/oder -aromen zusammenhängenden kolorimetrischen Prüfbeschreibungen berücksichtigt werden.

2. Übertragungsverfahren nach dem vorstehenden Anspruch, umfassend, vor dem ersten Lernschritt (60), einen Schritt des Festlegens (50), während dem Gewichte erster synaptischer Verbindungen, die Neuronen des neuronalen Netzes (1) zwei für zwei verbinden, festgelegt werden, sodass die den ersten synaptischen Verbindungen zugewiesenen Gewichte beim Lernschritt nicht verändert werden können.

3. Übertragungsverfahren nach dem vorstehenden Anspruch, wobei der Festlegungsschritt (50) von ersten Korrelationskoeffizienten ausgeht, wobei jeder erste Korrelationskoeffizient zwischen einem Molekül (Mi) der Gruppe von Molekülen und einer Farbe der Gruppe von Farben (Cj) berechnet wird, die in einem vorgeschalteten Schritt (40), von physikalisch-chemischen Prüfvektoren und kolorimetrischen Beschreibungen (DCt) ausgehend, die den Prüfgerüchen und/oder -aromen (Ot) zugeordnet sind, denen die physikalisch-chemischen Vektoren zugeordnet werden, berechnet werden.

4. Übertragungsverfahren nach dem vorstehenden Anspruch, wobei der vorgeschaltete Schritt (40) für mindestens eine Indexfarbe j (Cj) umfasst:

- einen Schritt des Auswählens (401) einer Teilgruppe (SEj) aus der Gruppe von Prüfgerüchen und/oder -aromen (Ot), die mit kolorimetrischen Prüfbeschreibungen (DCt), die für die Indexfarbe j Anteile ungleich Null aufweisen,
- einen ersten Schritt (402) des Zusammenfassens von Prüfgerüchen und/oder -aromen der Teilgruppe (SEj) in einem Gruppensatz von Prüfgerüchen und/oder Aromen, so dass es zwischen den physikalisch-chemischen Vektoren (PQt), die den Prüfgerüchen und/oder -aromen in einer Gruppe zugeordnet sind, größere erste Korrelationskoeffizienten gibt, als mit den physikalisch-chemischen Vektoren (PQt) die den in den anderen Gruppen des Gruppensatzes enthaltenen Prüfgerüchen und/oder - aromen zugeordnet sind,
- und, für mindestens eine dieser Gruppen,
- einen Schritt (403) des Erstellens einer Liste mit gemeinsamen Molekülen, die den Molekülen entsprechen, deren Prüfanteil bei allen physikalisch-chemischen Vektoren, die den Prüfgerüchen und/oder -aromen in der Gruppe zugeordnet sind, ungleich Null ist,
- und, für mindestens ein Molekül der Liste der gemeinsamen Moleküle,
- einen Schritt (404), bei dem eine erste Reihe (SU1) festgelegt wird, in der die Prüfgerüche und/oder -aromen in der Gruppe in aufsteigender Reihenfolge des Anteils des Moleküls in den physikalisch-chemischen Prüfvektoren, die den Prüfgerüchen und/oder -aromen zugeordnet sind, aufgeführt sind,
- einen Schritt (405), bei dem eine zweite Reihe (SU2) festgelegt wird, in der die Prüfgerüche und/oder -aromen in der Gruppe in aufsteigender Reihenfolge des Anteils der Farbe in den kolorimetrischen Beschreibungen, die den Prüfgerüchen und/oder -aromen zugeordnet sind, aufgeführt sind,
- einen Schritt (406) des Berechnens eines weiteren Korrelationskoeffizienten zwischen der ersten Reihe (SU1) und der zweiten Reihe (SU2),
- einen Schritt (407) des Berechnens mindestens eines ersten Korrelationskoeffizienten zwischen einem Molekül der Gruppe von Molekülen und einer Farbe der Gruppe von Farben aufgrund der anderen Korrelationskoeffizienten, die für das jeweilige Molekül und die jeweilige Farbe berechnet wurden.

5. Übertragungsverfahren nach einem der vorstehenden Ansprüche, wobei die kolorimetrische Beschreibung (DC) die theoretische kolorimetrische Beschreibung (DCT) ist.

6. Übertragungsverfahren nach einem der Ansprüche 1 bis 4, wobei der Schritt des Zuordnens (70) der kolorimetrischen Beschreibung umfasst: einen Schritt des Verbesserns (80) der theoretischen kolorimetrischen Beschreibung aufgrund von Werten einer Gruppe von sensorischen Beschreibungen, die dem jeweiligen mindestens einen Geruch bzw. Aroma zugeordnet werden, um die kolorimetrische Beschreibung zu erhalten, wobei die Gruppe von sensorischen Beschreibungen mindestens eine sensorische Beschreibung umfasst, die aus der nachfolgenden Gruppe gewählt ist: eine Quelle eines Geruchs bzw. Aromas, ein Genießbarkeitsindex, ein Annehmlichkeitsindex, ein Vertrautheitsindex, ein Aromaprofil, ein Intensitätsindex.

7. Übertragungsverfahren nach dem vorstehenden Anspruch, wobei der Schritt des Verbesserns (80) umfasst:

- einen Schritt (81) des Unterziehens mindestens eines zweiten künstlichen neuronalen Netzes des Indexes b einem zweiten Lernschritt, wobei ein zweites neuronales Netz eine mehrschichtige Architektur aufweist, die eine Eingangsschicht umfasst, die zum Empfang von Werten, die der Gruppe von sensorischen Beschreibungen zugeordnet sind, und am Ausgang erste Lm, zweite cm und dritte Hpm gemittelte Koordinaten ausgibt, wobei p eine Ganzzahl von 1 bis v in einem L, C, H genannten kolorimetrischen Bezugsrahmen ist, wobei das zweite neuronale Netz im zweiten Lernschritt Werte der Gruppe von sensorischen Beschreibungen, die den Prüfgerüchen und/oder -aromen zugeordnet sind, die in einer Farbreihe, die aus mindestens einem Teil der Prüfgerüche und/oder -aromen der Gruppe der Prüfgerüche und/oder -aromen besteht, empfängt,
- einen zweiten Schritt des Analysierens (82) des Geruchs bzw. Aromas unter Zuordnung des Indexes b zu den Werten der Gruppe von sensorischen Beschreibungen zum Eingang des zweiten neuronalen Netzes, das am Ausgang erste Lmbutb, zweite cmbutb und dritte Hpmbutb gemittelte Zielkoordinaten des Indexes b im Bezugsrahmen L, H, C einer dem betreffenden Geruch bzw. Aroma zugeordneten kolorimetrischen Beschreibung ausgibt,
- einen Schritt des Berechnens (83) einer neuen kolorimetrischen Beschreibung, die die neuen Anteile $qcnb_j$ umfasst, die für jeden Wert j zwischen 1 und J folgende Gleichungen erfüllen:

$$Lmbutb = \sum_{\substack{j=1 \\ J}}^{J} Lj * qcnb_j,$$

$$cmbutb = \sum_{j=1} cj * qcnb_j$$

$$Hpmbutb = \sum_{j=1}^{J} PHpj * qcnb_j$$

wobei p eine Ganzzahl von 1 bis v ist und v eine Ganzzahl ist, wobei Lj, cj und PHpj die jeweiligen Koordinaten der Farbe Cj des Indexes j im Bezugsrahmen L, H, C sind,
die für mindestens eine Farbe Cj den absoluten Betrag $|qcnb_j - qcTb_j|$ des Unterschieds zwischen dem neuen Anteil der Ordnung b und dem theoretischen Anteil der Ordnung b minimieren.

8. Verfahren nach einem der Ansprüche 6 bis 7, umfassend, vor dem Schritt des Verbesserns (80), einen zweiten Schritt des Zusammenfassens (72) von Prüfgerüchen und/oder -aromen, die zur Gruppe der Prüfgerüche und/oder -aromen (Ot) in einer Gruppe von sogenannten Farbreihen gehören, aufgrund der Ähnlichkeit der ihnen zugeordneten kolorimetrischen Prüfbeschreibungen (DCt).

9. Verfahren nach dem vorstehenden Anspruch, umfassend:

- einen Schritt des Berechnens von Ähnlichkeitskoeffizienten (73) zwischen dem Geruch und den jeweiligen Farbreihen aufgrund der Werte der Gruppe der dem Geruch bzw. Aroma und den Prüfgerüchen und/oder -aromen der jeweiligen Farbserien zugeordneten sensorischen Beschreibungen und/oder aufgrund der theoretischen kolorimetrischen Beschreibung und den den Prüfgerüchen der jeweiligen Geruchsreihen zugeordneten theoretischen kolorimetrischen Beschreibungen,
- einen Schritt des Verifizierens (74), bei dem unter den Farbreihen ähnliche Farbreihen erkannt werden, mit denen der Geruch bzw. das Aroma ein erstes vorgegebenes Ähnlichkeitskriterium erfüllt, und bei dem geprüft wird, ob der Geruch bzw. das Aroma ein zweites Ähnlichkeitskriterium mit einer Farbreihe aus den ähnlichen Farbreihen erfüllt,
- und wobei, wenn der Geruch bzw. das Aroma das erste und zweite Ähnlichkeitskriterium erfüllt, im zweiten Schritt des Analysierens (82) die Werte der Gruppe der dem Geruch bzw. Aroma zugeordneten sensorischen Beschreibungen einem einzigen zweiten neuronalen Netz zuführt, das im Voraus dem Lernschritt (81) unterzogen wurde, bei dem ihm am Eingang die Werte der Gruppe der den Prüfgerüchen und/oder -aromen der Farbserie zugeordneten sensorischen Beschreibungen, mit denen der Geruch bzw. das Aroma das zweite Ähnlichkeitskriterium erfüllt, eingibt,
- und wobei, wenn der Geruch bzw. das Aroma das erste, aber nicht das zweite Ähnlichkeitskriterium erfüllt, im zweiten Schritt des Analysierens (82) die Werte der Gruppe der dem Geruch bzw. Aroma zugeordneten sensorischen Beschreibungen einer Mehrzahl von zweiten neuronalen Netzen zuführt, die im Voraus dem Lernschritt (81) unterzogen wurde, bei der ihr am Eingang die Werte der Gruppe der den Prüfgerüchen und/oder -aromen der Farbserie zugeordneten sensorischen Beschreibungen in den Farbserien, mit denen der Geruch bzw. das Aroma das erste Ähnlichkeitskriterium erfüllt, eingibt, wobei die den jeweiligen Farben zugeordneten kolorimetrischen Anteile $(qc_j)$ lineare Kombinationen der den jeweiligen Farben zugeordneten neuen kolorimetrischen Anteile sind.

10. Verfahren nach einem der Ansprüche 6 bis 9, umfassend, vor dem Schritt des Verbesserns (80) einen Schritt des Korrigierens (75), der darin besteht, die theoretische kolorimetrische Beschreibung (DCT) anhand der Werte der Gruppe der dem Geruch bzw. Aroma zugeordneten Beschreibungen und aufgrund einer globalen Regel, die für mindestens eine Farbe die Berechnung der Wahrscheinlichkeit ihres Vorhandenseins in einer diesen Werten zugeordneten kolorimetrischen Beschreibung ermöglicht und anhand eines der Farbe zugeordneten kolorimetrischen Anteils zu aktualisieren, wobei der Schritt des Korrigierens (75) gefolgt wird durch die Rückkehr zum Schritt des Berechnens (73) der Ähnlichkeitskoeffizienten.

11. Verfahren nach einem der vorstehenden Ansprüche, wobei der Schritt (30) des Zuordnens einer kolorimetrischen Beschreibung, die eine Gruppe von jeweiligen Farben (Cj) zugeordnete Prüfanteile $(qct_j)$ umfasst, zu den Prüfgerüchen und/oder -aromen, umfasst:

- einen Schritt des Zuordnens (30a) von sog. Gutachtenfarben zu den Prüfgerüchen und/oder -aromen (Ot) durch eine Gruppe von menschlichen Gutachtern,
- einen Schritt des Erstellens (30b) einer kolorimetrischen sog. Prüfbeschreibung jedes Prüfgeruchs bzw. -aromas (Ot), umfassend Prüfanteile ($qct_j$), die einer Gruppe von jeweiligen Farben (Cj) zugeordnet sind, aufgrund des Anteils der Gutachter aus einer anderen Gruppe von Gutachtern, die alle oder einige der Gruppe von menschlichen Gutachtern umfasst, die jede der Gutachtenfarben dem Prüfgeruch bzw. - aroma (Ot) zugeordnet haben.

12. Übertragungsverfahren nach dem vorstehenden Anspruch, wobei die Prüfanteile ($qct_j$) der unterschiedlichen Farben (Cj) sich nach dem Anteil der Gutachter der anderen Gruppe von Gutachtern, die jede der Gutachtenfarben dem Aroma bzw. Geruch zugeordnet haben, und nach rechnerisch ermittelten Nähekoeffizienten zwischen der Gutachtenfarbe und den Farben der Gruppe von Farben bestimmen.

13. Übertragungsverfahren nach einem der vorstehenden Ansprüche, umfassend einen Schritt des Darstellens (90) des neuen Geruchs bzw. Aromas (O) mittels einer Farbkarte, in der der von einer Farbe (Cj) der Gruppe von Farben besetzte Prozentanteil der Fläche der Farbkarte, dem Verhältnis zwischen dem der Farbe zugeordneten Anteil ($qc_j$) an der kolorimetrischen Beschreibung und der Summe der einer Teilgruppe der Gruppe von Farben in der kolorimetrischen Beschreibung zugeordneten Anteile entspricht, sodass diese Summe der den von ihr umfassten Farben zugeordneten Anteile an der kolorimetrischen Beschreibung mindestens gleich einem vorgegebenen Schwellenwert ist.

14. Verfahren zum Übertragen einer ersten kolorimetrischen Beschreibung, umfassend erste kolorimetrische Anteile, die sich auf eine Gruppe von jeweiligen Farben in einer physikalisch-chemischen Beschreibung eines Ergebnisses beziehen, umfassend eine vorgegebene Gruppe von flüchtigen Molekülen, umfassend die Schritte:

   - einen Schritt des Durchführens einer physikalisch-chemischen Analyse einer Gruppe von sog. Prüfgerüchen und -aromen, einer physikalisch-chemischen Prüfbeschreibung, die einen sog. physikalisch-chemischen Prüfvektor, umfassend Prüfanteile, die mit der vorgegebenen Gruppe von jeweiligen flüchtigen Molekülen zusammenhängen,
   - einen ersten Schritt des Zuordnens jeweiliger kolorimetrischer Prüfbeschreibungen zu den Prüfgerüchen und/oder -aromen, umfassend sog. Prüfanteile, die mit einer Gruppe von jeweiligen Farben zusammenhängen,
   - einen zweiten Schritt des Zuordnens einer hypothetischen kolorimetrischen Beschreibung, umfassend hypothetische kolorimetrische Anteile an der Gruppe der jeweiligen Farben zu einem hypothetischen physikalisch-chemischen Vektor, umfassend hypothetische Anteile, die der Gruppe der jeweiligen flüchtigen Moleküle zugeordnet sind, wobei der zweite Zuordnungsschritt mittels eines ersten künstlichen neuronalen Netzes mit mehrschichtiger Architektur dadurch erfolgt, dass ihm am Eingang ein hypothetischer physikalisch-chemischer Vektor, umfassend hypothetische Anteile, die der Gruppe der jeweiligen flüchtigen Moleküle zugeordnet sind, präsentiert wird, wobei das erste neuronale Netz am Ausgang die hypothetische kolorimetrische Beschreibung erzeugt, wobei das erste neuronale Netz im Voraus einem ersten Lernschritt unterzogen wird, bei dem man ihm am Eingang physikalisch-chemische Vektoren, die mit der Gruppe von Prüfgerüchen und/oder -aromen zusammenhängen, zuführt, wobei der erste Lernschritt unter Aufsicht erfolgt und dabei die mit der Gruppe der jeweiligen Prüfgerüche und/oder -aromen zusammenhängenden kolorimetrischen Prüfbeschreibungen berücksichtigt werden,
   - einen Fehlerberechnungsschritt, bei dem ein Fehler berechnet wird, der eine Abweichung zwischen der hypothetischen kolorimetrischen Beschreibung und der ersten kolorimetrischen Beschreibung darstellt, wobei dem Fehlerberechnungsschritt ein Schritt des Aktualisierens des hypothetischen physikalisch-chemischen Vektors aufgrund des Fehlers und des hypothetischen physikalisch-chemischen Vektors folgt und zum Zuordnungsschritt zurückgekehrt wird, wenn der Fehler eine vorgegebene Fehlerschwelle übersteigt, wobei sich die resultierende physikalisch-chemische Beschreibung nach dem hypothetischen physikalisch-chemischen Vektor bestimmt, der einen Rechenfehler kleiner oder gleich der Fehlerschwelle ergibt.

15. Übertragungsverfahren nach dem vorstehenden Anspruch, wobei die erste Beschreibung sog. ersten Werten einer Gruppe von sensorischen Beschreibungen zugeordnet ist und die Prüfgerüche und/oder -aromen Werten der Gruppe von sensorischen Beschreibungen zugeordnet sind, wobei die Gruppe der sensorischen Beschreibungen mindestens eine sensorische Beschreibung umfasst, die aus der nachfolgenden Gruppe gewählt ist: eine Quelle eines Geruchs bzw. Aromas, ein Genießbarkeitsindex, ein Annehmlichkeitsindex, ein Vertrautheitsindex, ein Aromaprofil, ein Intensitätsindex, und umfassend einen vorgeschalteten Schritt des Bestimmens des hypothetischen physikalisch-chemischen Vektors aufgrund sog. erster Werte der Gruppe der sensorischen Beschreibungen und der Werte der Gruppe der dem Prüfgeruch bzw. - aroma zugeordneten sensorischen Beschreibungen.

**16.** Übertragungsverfahren nach dem vorstehenden Anspruch, umfassend, vor dem Schritt des Bestimmens des hypothetischen physikalisch-chemischen Vektors:

- einen Schritt des Konstruierens einer weiteren globalen Regel, die es ermöglicht, aufgrund der Werte einer Gruppe von sensorischen Beschreibungen, die einem Geruch bzw. Aroma zugeordnet sind, zu bestimmen, ob die Wahrscheinlichkeit des Vorhandenseins der Gruppe der jeweiligen Moleküle im Geruch bzw. Aroma O ungleich Null ist, wobei dieser Schritt aufgrund der physikalisch-chemischen Prüfvektoren und der Werte der Gruppe von sensorischen Beschreibungen, die den Prüfgerüchen und/oder - aromen zugeordnet sind, erfolgt,
- einen Schritt des Zusammenfassens der Prüfgerüche und/oder -Aromen in einer Gruppe von Farbreihen nach der Ähnlichkeit ihrer jeweiligen kolorimetrischen Prüfbeschreibungen,

wobei der Schritt des Bestimmens des hypothetischen physikalisch-chemischen Vektors umfasst:

- einen Schritt des Bestimmens der Wahrscheinlichkeit des Vorhandenseins der Gruppe der jeweiligen Moleküle im resultierenden Geruch bzw. Aroma, wobei dieser Schritt aufgrund der ersten Werte der Gruppe der sensorischen Beschreibungen und der anderen globalen Regel erfolgt,
- einen Schritt des Erstellens einer Liste von möglichen Molekülen aus der Gruppe der Moleküle, wobei diese Liste der möglichen Moleküle den Molekülen der Gruppe der Moleküle entspricht, bei denen die Wahrscheinlichkeit ihres Vorhandenseins ungleich Null ist,
- einen Schritt des Berechnens von Korrelationskoeffizienten zwischen der ersten Beschreibung und den jeweiligen Farbreihen aufgrund der ersten Werte der Gruppe der sensorischen Beschreibungen und der Werte der Gruppe der sensorischen Beschreibungen, die den jeweiligen Prüfgerüchen und/oder -aromen zugeordnet sind, sowie aufgrund der ersten kolorimetrischen Beschreibungen und der kolorimetrischen Prüfbeschreibungen,
- einen Schritt des Verifizierens, bei dem unter den Farbreihen ähnliche Farbreihen erkannt werden, mit denen die erste Beschreibung ein vorgegebenes Ähnlichkeitskriterium erfüllt, aufgrund der im vorigen Absatz erwähnten Ähnlichkeitskoeffizienten,
- einen Schritt des Erstellens des hypothetischen physikalisch-chemischen Vektors aufgrund der Liste der möglichen Moleküle und der physikalisch-chemischen Prüfvektoren, die den Prüfgerüchen und/oder -aromen in den Farbreihen, mit denen die erste Beschreibung das Korrelationskriterium erfüllt, zugeordnet sind, wobei der hypothetische physikalisch-chemische Vektor hypothetische Anteile umfasst, die den jeweiligen möglichen Molekülen Mi zugeordnet sind.

**17.** Verfahren nach einem der Ansprüche 14 bis 16, wobei folgende Schritte von einem Rechner ausgeführt werden: der zweite Schritt des Zuordnens einer kolorimetrischen Beschreibung, der erste Lernschritt, der Fehlerberechnungsschritt, der Schritt des Aktualisierens des physikalisch-chemischen Vektors.

**Claims**

**1.** A method for transcribing an odor or an aroma (O) into a colorimetric description comprising the following steps:

- a first step (10) of physico-chemical analysis of said odor or of said aroma (O) in order to associate with it a physico-chemical description (PCN) comprising a physico-chemical vector (PQ), comprising proportions ($qm_i$) associated with a predetermined set of respective volatile molecules (Mi),
- a second step (20) of physico-chemical analysis of a set of so-called test odors and/or aromas (Ot) in order to assign to each test odor or aroma (Ot) of said set a so-called test physico-chemical description ($PC_t$) comprising a so-called test physico-chemical vector ($PQ_t$) comprising test proportions ($qmt_i$) associated with said predetermined set of respective volatile molecules (Mi),
- a step (30) of assigning to the test odors and/or aromas (Ot) respective test colorimetric descriptions (DCt) comprising so-called test proportions ($qct_j$) associated with a set of respective colors (Cj),
- a step (70) of assigning to the odor or to the aroma (O) a colorimetric description comprising proportions ($qc_j$) associated with said respective colors (Cj), said assignment step (70) comprising a first step (71) of assigning to the odor or to the aroma a theoretical colorimetric description (DCT) comprising theoretical colorimetric proportions ($qcT_j$) associated with said respective colors (Cj), this first assignment step (71) being performed by means of a first artificial neural network (1) exhibiting a layered architecture, by presenting to it, as input, the physico-chemical vector (PQ), said first neural network (1) generating, as output, said theoretical colorimetric description (DCT), said first neural network (1) being previously subjected to a first learning step (60) during which there are supplied to it, as input, physico-chemical vectors (PQt) associated with the set of test odors and/or aromas, said first learning step being performed in a supervised manner by taking into account the test colorimetric descriptions associated with the respective set of test odors and/or aromas.

2. The transcription method as claimed in the preceding claim, comprising, prior to the first learning step (60), a fixing step (50), during which weights of first synaptic links linking neurons of said neural network (1) two by two, are fixed such that the weights associated with the first synaptic links are not modifiable during the learning step.

3. The transcription method as claimed in the preceding claim, in which the fixing step (50) is performed from first correlation coefficients, each first correlation coefficient being computed between a molecule (Mi) of the set of molecules and a color of the set of colors (Cj), computed in a preliminary step (40) from test physico-chemical vectors and colorimetric descriptions (DCt) associated with the test odors and/or aromas (Ot) to which said physico-chemical vectors are assigned.

4. The transcription method as claimed in the preceding claim, in which the preliminary step (40) comprises for at least one color of index j (Cj):

- a step (401) of selecting, from the set of test odors and/or aromas (Ot), a subset (SEj) of index j of test odors and/or aromas which are associated with test colorimetric descriptions (DCt) exhibiting non-zero proportions for said color of index j,
- a first step (402) of grouping together test odors and/or aromas of the subset (SEj) in a set of groups of test odors and/or aromas, such that the physico-chemical vectors (PQt) associated with the test odors and/or aromas present in a group exhibit, between them, greater first correlation coefficients than with the physico-chemical vectors (PQt) associated with the test odors and/or aromas present in the other groups of said set of groups,
- and for at least one of said groups,
- a step (403) of preparing a list of common molecules corresponding to the molecules for which the test proportion is non-zero in all the test physico-chemical vectors associated with the test odors and/or aromas grouped together in said group,
- and, for at least one molecule taken from the list of common molecules,
- a step (404) during which a first series (SU1) is established, in which the test odors and/or aromas grouped together in said group are arranged in ascending order of the proportion of said molecule in the test physico-chemical vectors associated with said test odors and/or aromas,
- a step (405) during which a second series (SU2) is established, in which the test odors and/or aromas grouped together in said group are arranged in ascending order of the proportion of said color in the colorimetric descriptions associated with said test odors and/or aromas,
- a step (406) of computing another correlation coefficient between the first series (SU1) and the second series (SU2),
- a step (407) of computing at least one first correlation coefficient between a molecule of the set of molecules and a color of the set of colors from the other correlation coefficients computed for said molecule and said color.

5. The transcription method as claimed in any one of the preceding claims, in which the colorimetric description (DC) is the theoretical colorimetric description (DCT).

6. The transcription method as claimed in any one of claims 1 to 4, in which the step (70) of assigning the colorimetric description comprises a step (80) of enhancing the theoretical colorimetric description from values of a set of sensory descriptors assigned to the odor or to the aroma and to respective test odors and/or aromas so as to obtain said colorimetric description, the set of sensory descriptors comprising at least one sensory descriptor taken from a source of an odor or of an aroma, a wholesomeness index, an agreeability index, a familiarity index, an olfactory note, and an intensity index.

7. The transcription method as claimed in the preceding claim, in which the enhancement step (80) comprises:

- a step (81) of submitting at least one second artificial neural network of index b to a second learning step, a second neural network exhibiting a layered architecture comprising an input layer suitable for receiving values associated with the set of sensory descriptors and supplying as output first Lm, second cm and third Hpm mean coordinates, in which p is an integer ranging from 1 to v an integer, in a colorimetric reference frame called L, C, H, said second neural network receiving, during the second learning step, values of the set of sensory descriptors associated with the test odors and/or aromas present in a color series made up of at least a part of the test odors and/or aromas of the set of test odors and/or aromas,
- a second step (82) of analysis of the odor or of the aroma by supplying the values of the set of sensory descriptors as input for said second neural network of index b which supplies, as output, first Lmbutb, second cmbutb and third Hpmbutb target mean coordinates of index b in reference frame L, H, C, of a target colorimetric description associated with the odor or with the aroma concerned,
- a step (83) of computing a new colorimetric description comprising new proportions $qcnb_j$ which observe, regardless of the value of j between 1 and J, the following equations:

$$Lmbutb = \sum_{j=1}^{J} Lj * qcnb_j$$

$$cmbutb = \sum_{j=1}^{J} cj * qcnb_j \ ,$$

$$Hpmbutb = \sum_{j=1}^{J} PHpj * qcnb_j$$

with p an integer ranging from 1 to v and v an integer, in which Lj, cj and PHpj are the respective coordinates of the color Cj of index j in the reference frame L, H, C,
and which minimize, for at least one color Cj, the absolute value $|qcnb_j - qcTb_j|$ of the difference between the new proportion of order b and the theoretical proportion of order b.

8. The transcription method as claimed in any one of claims 6 to 7, comprising, prior to the enhancement step (80), a second step (72) of grouping together test odors and/or aromas belonging to the set of test odors and/or aromas (Ot) in a set of series, called color series, by similarity of the test colorimetric descriptions (DCt) which are assigned to them.

9. The transcription method as claimed in the preceding claim, comprising:

- a step (73) of computing coefficients of similarity between the odor and the respective color series, from values of the set of sensory descriptors assigned to the odor or to the aroma and to the test odors and/or aromas grouped together in respective color series and/or from the theoretical colorimetric description and from the test colorimetric descriptions assigned to the test odors grouped together in the respective series of odors,
- a verification step (74) during which there are identified, from the color series, similar color series with which the odor or the aroma satisfies a first predetermined similarity criterion, and during which the question of whether the odor or the aroma satisfies a second criterion of similarity with a color series taken from the similar color series is verified,
- and, when the odor or the aroma satisfies the first and the second similarity criteria, the values of the set of sensory descriptors assigned to the odor or to the aroma are subjected, during the second analysis step (82), to a single second neural network previously subjected to the learning step (81) during which the values of the set of sensory descriptors assigned to the test odors and/or aromas grouped together in said color series with which the odor or the aroma satisfies the second similarity criterion are supplied to it as input,
- and, when the odor or the aroma satisfies the first similarity criterion but not the second similarity criterion, the values of the set of sensory descriptors assigned to the odor or to the aroma are subjected, during the second analysis step (82), to a plurality of second neural networks previously subjected to the learning step (81) during which there are supplied to it, as input, the values of the set of sensory descriptors assigned to the test odors and/or aromas grouped together in the color series with which the odor or the aroma satisfies the first similarity criterion, the colorimetric proportions ($qc_j$) associated with said respective colors being linear combinations of new colorimetric proportions associated with the respective colors.

10. The transcription method as claimed in any one of claims 6 to 9, comprising, prior to the enhancement step (80), a correction step (75) consisting in updating the theoretical colorimetric description (DCT) from the values of the set of descriptors associated with the odor or with the aroma and from a global rule making it possible to compute, for at least one color, and from values of the set of sensory descriptors assigned to the aroma or to the odor, a probability of presence, in a colorimetric description associated with these values, of a colorimetric proportion associated with said color, said correction step (75) being followed by a return to the step (73) of computing similarity coefficients.

11. The transcription method as claimed in any one of the preceding claims, in which the step (30) of assigning to the test odors and/or aromas a colorimetric description comprising test proportions ($qct_j$) associated with a set of respective colors (Cj), comprises:

- a step (30a) of assigning colors, called judged colors, to test odors and/or aromas (Ot) by a set of human judges,
- a step (30b) of preparing a so-called test colorimetric description of each test odor or aroma (Ot) comprising test proportions ($qct_j$) associated with a set of respective colors (Cj) from the proportion of judges who, out of the judges belonging to another set of judges comprising all or part of said set of human judges, have each assigned judged colors to said test odor or aroma (Ot).

12. The transcription method as claimed in the preceding claim, in which the test proportions ($qct_j$) relating to the different colors ($C_j$) are determined from the proportion of judges who, out of the judges belonging to the other set of judges, have each assigned judged colors to said aroma or to said odor and from proximity coefficients computed between the judged color and the colors of the set of colors.

13. The transcription method as claimed in any one of the preceding claims, comprising a step (90) of representing the new odor or the new aroma (O) by means of a chromatic map in which the percentage of the surface of the chromatic map occupied by a color (Cj) of the set of colors corresponds to the ratio between the proportion associated with said color ($qc_j$) in the colorimetric description and the sum of the proportions associated with a subset of the set of colors in the colorimetric description, the subset being chosen in such a way that the sum of the proportions associated with the colors that it contains, in the colorimetric description, is at least equal to a predetermined threshold.

14. A method for transcribing an initial colorimetric description comprising initial colorimetric proportions relating to a set of respective colors into a result physico-chemical description comprising a list of molecules comprising the following steps:

- a step of physico-chemical analysis of a set of so-called test odors and/or aromas in order to assign, to each test odor or aroma of said set, a so-called test physico-chemical description comprising a so-called test physico-chemical vector comprising test proportions associated with said predetermined set of respective volatile molecules,
- a first step of assigning to the test odors and/or aromas respective test colorimetric descriptions comprising so-called test proportions associated with a set of respective colors,
- a second step of assigning of a hypothetical colorimetric description comprising hypothetical colorimetric proportions to the set of respective colors, to a hypothetical physico-chemical vector comprising hypothetical proportions associated with said set of respective volatile molecules, this second assignment step being performed by means of a first artificial neural network exhibiting a layered architecture, by presenting to it, as input, a hypothetical physico-chemical vector comprising hypothetical proportions associated with said set of respective volatile molecules, said first neural network generating, as output, said hypothetical colorimetric description, said first neural network being previously subjected to a first learning step during which there are supplied to it, as input, physico-chemical vectors associated with the set of test odors and/or aromas, said first learning step being performed in a supervised manner by taking into account the test colorimetric descriptions associated with the set of respective test odors and/or aromas,
- an error computation step in which an error representative of a deviation between the hypothetical colorimetric description and the initial colorimetric description is computed, said error computation step being followed by a step of updating the hypothetical physico-chemical vector, from the error and the hypothetical physico-chemical vector, and of return to the assignment step as long as the error is greater than a predetermined error threshold, the result physico-chemical description being determined from the hypothetical physico-chemical vector which generates a computation error less than or equal to the error.

15. The transcription method as claimed in the preceding claim, in which the initial description is associated with so-called initial values of a set of sensory descriptors and the test odors and/or aromas are associated with values of said set of sensory descriptors, said set of sensory descriptors comprises at least one sensory descriptor taken from a source of an odor or of an aroma, a wholesomeness index, an agreeability index, a familiarity index, an olfactory note and an intensity index, and comprising a preliminary step of determining the hypothetical physico-chemical vector from so-called initial values of the set of sensory descriptors and from values of said set of sensory descriptors associated with the test odor or aroma.

**16.** The transcription method as claimed in the preceding claim, comprising, prior to the step of determining the hypothetical physico-chemical vector:

- a step of constructing another global law making it possible to determine, from the values of a set of sensory descriptors associated with an odor or an aroma, the probabilities of presence of the set of respective molecules in the odor or the aroma O being non-zero, this step being performed from test physico-chemical vectors and from the values of the set of sensory descriptors associated with the test odors and/or aromas,
- a step of grouping together the test odors and/or aromas in a set of color series by similarity of their respective test colorimetric descriptions,

the step of determining the hypothetical physico-chemical vector comprising:

- a step of determining probabilities of presence, in the result odor or aroma, of the set of the respective molecules, this step being performed from the initial values of the set of sensory descriptors and from the other global law,
- a step of establishing a list of possible molecules taken from the set of molecules, this list of possible molecules corresponding to the molecules which, out of the molecules of the set of molecules, exhibit a non-zero probability of presence,
- a step of computing correlation coefficients between the initial description and the respective color series from initial values of the set of sensory descriptors and from the values of the set of sensory descriptors associated with the respective test odors and/or aromas as well as from the test and initial colorimetric descriptions,
- a verification step during which there are identified, out of the color series, the color series with which the initial description satisfies a predetermined correlation criterion, this step is performed from similarity coefficients cited in the preceding paragraph,
- a step of preparing the hypothetical physico-chemical vector from the list of possible molecules and from test physico-chemical vectors associated with the test odors and/or aromas included in the color series with which the initial description satisfies the correlation criterion, the hypothetical physico-chemical vector comprising hypothetical proportions associated with the respective possible molecules Mi.

**17.** The method as claimed in any one of claims 14 to 16, in which the following steps are implemented by a computer: the second step of assigning a colorimetric description, the first learning step, the error computation step, the step of updating the physico-chemical vector.

10 ⌐

| Première étape<br>d'analyse<br>physiquo-chimique d'O |
|---|

20 ⌐

| Première étape<br>d'analyse<br>physiquo-chimique des Ot |
|---|

30 ⌐

| Attribution, aux Ot, de<br>descriptions<br>colorimétriques test |
|---|

$PC_t : \overrightarrow{PQ_t}$ (qmt$_i$) i=1 à N
t= 1à T

$PC : \overrightarrow{PQ}$ (qm$_i$) i=1 à N

$DC_t$ : (qct$_j$) j=1 à J
t= 1à T

40 ⌐

| Etape préalable de calcul<br>de premiers coefficients<br>de corrélation |
|---|

50 ⌐

| Fixation de poids de<br>liaisons synaptiques<br>du 1$^{er}$ réseau de neurones |
|---|

60 ⌐

| Première étape<br>d'apprentissage du 1$^{er}$<br>réseau de neurones |
|---|

70 ⌐

| Attribution d'une<br>description<br>colorimétrique à O |
|---|

90 ⌐

| Représentation |
|---|

FIG.1a

de 60

71 — Première étape d'attribution d'une description colorimétrique théorique

$DCT = (qcT_j)\,j=1\ à\ J$

72 — Regroupement des Ot dans des séries couleurs

73 — Calcul de coefficients de similarité entre O et les séries couleurs

74 — Vérification du respect de C1 et C2

70

75 — Etape de correction de DCT

81 — Deuxième étape d'apprentissage

82 — Deuxième étape d'analyse

83 — Détermination d'une nouvelle description colorimétrique

80

DCN

84 — Détermination de la description colorimétrique à partir de DCN

FIG.1b

30

30a — Attribution de couleurs
à des Ot par un ensemble
initial de juges humains

Pour chaque Ot

30b — Elaboration d'une description
colorimétrique test

DCT : $(qcT_j)$ j=1 à J

# FIG.2

1

2 NE1    3 NI1    4 NS1

VE1      VI1      VS1

NEi      NIm      NSj

VEi      VIm      VSj      φ    → DCT : $(qcT_j)$ j=1 à J

NEN      NIM      NSJ

VEN      VIM      VSJ

# FIG.3

Pour au moins une Cj

```
Sélection d'un sous ensemble
SEj                                    401

Calcul d'un ensemble EC
de deuxièmes coefficients              402a
de corrélation

Création d'un ensemble de             402b
groupes d'Ot

Formation d'un nouvel ensemble        402c
NEC
```

402

402f

Génération de EC2
comprenant des
troisièmes coefficients de
corrélation C3

G1 ≠ G2

Identification de
G1 et G2            402d

EC ≠ φ

G1 = G2

EC = NEC            402e

Tous les C3 > S1

Fusion de
G1 et G2

EC = φ

402g

Pour au moins un des groupes obtenus :

```
Elaboration d'une liste de
molécules communes
```
403

```
Etablissement de SU1
```
404

```
Etablissement de SU2
```
405

```
Calcul d'un autre coefficient de
corrélation
```
406

```
Calcul des premiers
coefficients de corrélation
```
407

FIG.4

FIG.5

FIG.6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20090271003 A

- EP 1566633 A

**Littérature non-brevet citée dans la description**

- **YU-JIN KIM.** Can eyes smell ? Cross modal correspondence between color hue-tone and frangrance family. *Color Research & Application,* 31 Octobre 2011, vol. 38 (2), 139-156